# EUROPEAN PATENT SPECIFICATION

(11) **EP 1 755 604 B1**
(45) Date of publication and mention of the grant of the patent: **14.10.2009**
(21) Application number: 05742958.1
(22) Date of filing: 12.05.2005
(51) Int. Cl.: A61K 31/485, A61P 29/00, A61P 1/00, A61P 17/00, A61P 17/06, A61P 25/28, A61P 37/08, A61P 35/00, A61P 9/00, A61P 3/10, A61P 3/04, A61P 27/06, A61P 9/10

(54) **USE OF OPIOID RECEPTOR ANTAGONIST COMPOUNDS FOR THE PREVENTION AND/OR TREATMENT OF DISEASES ASSOCIATED WITH THE TARGET CALCINEURIN**
VERWENDUNG VON OPIOID-REZEPTORANTAGONISTEN ZUR PRÄVENTION UND/ODER BEHANDLUNG VON ERKRANKUNGEN IM ZUSAMMENHANG MIT DEM TARGET CALCINEURIN
UTILISATION D'ANTAGONISTES AUX RÉCEPTEURS OPIOIDES POUR PRÉVENIR ET/OU TRAITER LES MALADIES ASSOCIÉES À LA CIBLE CALCINEURINE

(30) Priority: 12.05.2004 EP 04011293
(43) Date of publication of application: 28.02.2007
(73) Proprietor: ALCASYNN PHARMACEUTICALS GMBH, 6020 Innsbruck (AT)
(72) Inventor: SCHMIDHAMMER, Helmut, A-6020 Innsbruck (AT)
(74) Representative: Grünecker, Kinkeldey, Stockmair & Schwanhäusser Anwaltssozietät
(86) International application number: PCT/EP2005/005176
(87) International publication number: WO 2005/107752

(56) References cited:
- EP-A- 0 614 898
- EP-A- 0 614 898
- EP-A- 0 894 799
- WO-A-01/12195
- WO-A-95/31463
- WO-A-95/31463
- WO-A-20/04026819
- WO-A-20/04026819
- SPETEA M ET AL: "Binding, pharmacological and immunological profiles of the delta-selective opioid receptor antagonist HS 378." LIFE SCIENCES. 31 AUG 2001, vol. 69, no. 15, 31 August 2001 (2001-08-31), pages 1775-1782, XP008035572 ISSN: 0024-3205
- LAHSEN, JOAQUIN ET AL: "Structure-activity relationship study of nonpeptide .delta.-opioid receptor ligands" HELVETICA CHIMICA ACTA , 84(11), 3299-3305 CODEN: HCACAV; ISSN: 0018-019X, 2001, XP008035577
- DATABASE BIOSIS [Online] BIOSCIENCES INFORMATION SERVICE, PHILADELPHIA, PA, US; December 2003 (2003-12), DORTCH-CARNES JUANITA ET AL: "Delta-opioid agonist-stimulated inositol phosphate formation in isolated, rabbit iris-ciliary bodies: Role of Gi/o proteins and Gbetagamma-subunits." XP002460802 Database accession no. PREV200400162791 & EXPERIMENTAL EYE RESEARCH, vol. 77, no. 6, December 2003 (2003-12), pages 647-652, ISSN: 0014-4835
- SPETEA M ET AL: "Binding, pharmacological and immunological profiles of the delta-selective opioid receptor antagonist HS 378." LIFE SCIENCES. 31 AUG 2001, vol. 69, no. 15, 31 August 2001 (2001-08-31), pages 1775-1782, XP008035572 ISSN: 0024-3205
- LAHSEN, JOAQUIN ET AL: "Structure-activity relationship study of nonpeptide .delta.-opioid receptor ligands" HELVETICA CHIMICA ACTA , 84(11), 3299-3305 CODEN: HCACAV; ISSN: 0018-019X, 2001, XP008035577
- BIYASHEV, DAUREN ET AL: "Novel delta-opioid-receptor-selective ligands in the 14-alkoxy-substituted indolo- and benzofuromorphinan series" HELVETICA CHIMICA ACTA , 84(7), 2015-2021 CODEN: HCACAV; ISSN: 0018-019X, 2001, XP008035570

## Description

### Field of the invention

The present invention is related to the use of opioid receptor antagonists as well as their pharmaceutically acceptable salts for the manufacture of a medicament for the treatment of glaucoma and/or uveitis.

### Background of the invention

The further advance in medicinal research has yielded over the last decades a broad variety of powerful medicaments useful in a broad variety of indications. One important class of such compounds are opioid antagonists which are being used in the treatment of drug addiction and opioid induced constipation. Great improvements have been achieved, including the preparation of selective and long-lasting medicaments, showing furthermore a reduced tendency towards undesirable side-effects. However, other fields still require improvements, in particular chronic disorders and disorders affecting large parts of a given population. In this respect much attention has been focused on the developments of new classes of compounds showing a desired profile of activity concerning selected disorders.

However, the development of the understanding of the mechanisms of disorders on a molecular level has also greatly improved the knowledge as regards biological targets which appear to provide great promise concerning the treatment of larger groups of disorders, which all are interrelated with a given biological target.

In this respect the target calcineurin is an important target offering great promise for the treatments of a broad variety of disorders.

### Object of the present invention

Accordingly it is the object of the present invention to provide a group of compounds enabling the treatment of disorders by means of interaction with the target calcineurin, in order to enable the prevention and/or treatments of disorders such as glaucoma and/or uveitis.

### Outline of the invention

Accordingly the present invention provides the use of compounds of the formula (I) as defined below as defined in claims 1 and 7. Preferred embodiments are given below and defined in the subclaims, 2 to 6 and 8 to 12.

### COMPOUNDS OF FORMULA (I):

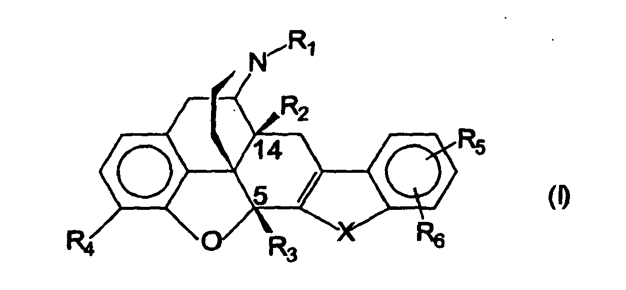

wherein
R₁ represents C₁-C₁₀ alkenyl; C₄-C₁₀ cycloalkylalkyl wherein the cycloalkyl is C₃-C₆ cycloalkyl and the alkyl is C₁-C₄ alkyl; C₄-C₁₀ cycloalkenylalkyl wherein the cycloalkenyl is C₃-C₆ cycloalkenyl and the alkyl is C₁-C₄ alkyl; C₇-C₁₆ arylalkyl wherein the aryl is C₆-C₁₀ aryl and the alkyl is C₁-C₆ alkyl; C₈-C₁₆ arylalkenyl wherein the aryl is C₆-C₁₀ aryl and the alkenyl is C₂-C₈ alkenyl;
R₂ represents hydrogen, hydroxy, C₁-C₆ alkoxy; C₁-C₆ alkenyloxy; C₇-C₁₆ arylalkyloxy wherein the aryl is C₆-C₁₀ aryl and the alkyloxy is C₁-C₆ alkyloxy; C₇-C₁₆ arylalkenyloxy wherein the aryl is C₆-C₁₀ aryl and the alkenyloxy is C₁-C₆ alkenyloxy; C₁-C₆ alkanoyloxy; C₇-C₁₆ arylalkanoyloxy wherein the aryl is C₆-C₁₀ aryl and the alkylaroyloxy is C₁-C₆ alkylaroyloxy;
R₃ represents hydrogen, C₁-C₆ alkyl; C₁-C₆ alkenyl; C₇-C₁₆ arylalkyl wherein the aryl is C₆-C₁₀ aryl and the alkyl is C₁-C₆ alkyl; C₇-C₁₆ arylalkenyl wherein the aryl is C₆-C₁₀ aryl and the alkenyl is C₁-C₆ alkenyl; hydroxy(C₁-C₆)alkyl; alkoxyalkyl wherein the alkoxy is C₁-C₆ alkoxy and the alkyl is C₁-C₆ alkyl; CO₂H; CO₂(C₁-C₆ alkyl);
R₄ is hydrogen, hydroxy; C₁-C₆ alkoxy; C₇-C₁₆ arylalkyloxy wherein the aryl is C₆-C₁₀ aryl and the alkyloxy is C₁-C₆ alkyloxy; C₁-C₆ alkenyloxy; C₁-C₆ alkanoyloxy; C₇-C₁₆ arylalkanoyloxy wherein the aryl is C₆-C₁₀ aryl and the alkanoyloxy is C₁-C₆ alkanoyloxy; C₂-C₁₀ alkyloxyalkoxy wherein alkyloxy is C₁-C₄ alkyloxy and alkoxy is C₁-C₆ alkoxy;
R₅ and R₆ each independently represent hydrogen; OH; C₁-C₆ alkoxy; C₁-C₆ alkyl; hydroxyalkyl wherein the alkyl is C₁-C₆ alkyl; halo; nitro; cyano; thiocyanato; trifluoromethyl; CO₂H; CO₂(C₁-C₆alkyl); CONH₂; CONH(C₁-C₆alkyl); CON(C₁-C₆ alkyl)₂; amino; C₁-C₆ monoalkyl amino; C₁-C₆ dialkyl amino, C₅-C₆ cycloalkylamino; SH; SO₃H; SO₃(C₁-C₆ alkyl); SO₂(C₁-C₆ alkyl); SO₂NH₂; SO₂NH(C₁-C₆ alkyl); SO₂NH (C₇-C₁₆ arylalkyl); SO(C₁-C₆ alkyl); or R₅ and R₆ together form a phenyl ring which may be unsubstituted or substituted by halo, nitro, cyano, thiocyanato; C₁-C₆ alkyl; trifluoromethyl; C₁-C₆ alkoxy, CO₂H, CO(C₁-C₆ alkyl), amino, C₁-C₆ monoalkylamino, C₁-C₆ dialkylamino, SH; SO₃H; SO₃(C₁-C₆ alkyl), SO₂(C₁-C₆ alkyl), SO(C₁-C₈ alkyl), and
X represents oxygen; sulfur; CH=CH or NR₉ wherein R₉ is H, C₁-C₆ alkyl, C₁-C₆ alkenyl, C₇-C₁₆ arylalkyl wherein the aryl is C₆-C₁₀ aryl and the alkyl is C₁-C₆ alkyl, C₇-C₁₆ arylalkenyl wherein the aryl is C₆-C₁₀ aryl and the alkenyl is C₁-C₆ alkenyl; C₁-C₆ alkanoyl,
with the proviso that when R₂ is hydroxy R₃ cannot be hydrogen, except when R₄ is hydrogen, OCH₂OCH₃, OCH₂OC₂H₅ or OC(Ph)₃;
and pharmacologically acceptable salts thereof.

Preferred embodiments of compounds of formula (I) are as follows: 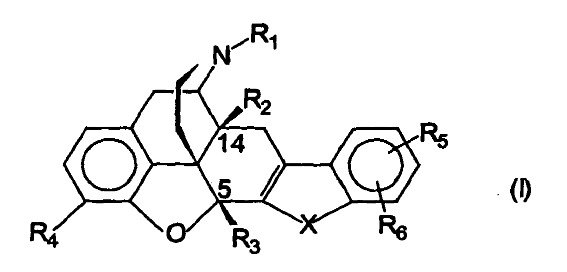 wherein
R₁ is: C₁-C₁₀-alkenyl; C₄-C₁₀-cycloalkylalkyl wherein cycloalkyl is C₃-C₆-cycloalkyl and alkyl is C₁-C₄-alkyl; C₄-C₁₀-cycloalkenylalkyl wherein cycloalkenyl is C₃-C₆-cycloalkenyl and alkyl is C₁-C₄-alkyl; C₇-C₁₆-arylalkyl wherein aryl is C₆-C₁₀-aryl and alkyl is C₁-C₆-alkyl; C₈-C₁₆-arylalkenyl wherein aryl is C₆-d₁₀-aryl and alkenyl is C₂-C₆-alkenyl;
R₂ is: C₁-C₆-alkoxy; C₁-C₆-alkenyloxy; C₇-C₁₆-arylalkyloxy wherein aryl is C₆-C₁₀-aryl and alkyloxy is C₁-C₆-alkyloxy; C₇-C₁₆-arylalkenyloxy wherein aryl is C₆-C₁₀-aryl and alkenyloxy is C₁-C₆-alkenyloxy;
R₃ is: hydrogen, C₁-C₆-alkyl; C₁-C₆-alkenyl; C₇-C₁₆-arylalkyl wherein aryl is C₆-C₁₀-aryl and alkyl is C₁-C₆-alkyl; C₇-C₁₆-arylalkenyl wherein aryl is C₆-C₁₀-aryl and alkenyl is C₁-C₆-alkenyl; hydroxy(C₁-C₆)alkyl; alkoxyalkyl wherein alkoxy is C₁-C₆-alkoxy and alkyl is C₁-C₆-alkyl ist; CO₂H; CO₂(C₁-C₆ alkyl);
R₄ is: hydroxy, C₁-C₆-alkoxy; C₇-C₁₆-arylalkyloxy wherein aryl is C₆-C₁₀-aryl and alkyloxy is C₁-C₆-alkyloxy; C₁-C₆-alkenyloxy; C₂-C₁₀-alkyloxyalkoxy wherein alkyloxy is C₁-C₄-alkyloxy and alkoxy is C₁-C₆-alkoxy;
R₅ and R₆ each independently represent: hydrogen; OH; C₁-C₆-alkoxy; C₁-C₆-alkyl; hydroxyalkyl wherein alkyl is C₁-C₆-alkyl; halogen; nitro; cyano; thiocyanato; trifluoromethyl; CO₂H; CO₂(C₁-C₆-alkyl); CONH₂; CONH(C₁-C₆-alkyl); CON(C₁-C₆-alkyl)₂; amino; C₁-C₆-monoalkylamino; C₁-C₆-dialkylamino; C₅-C₆-cycloalkylamino; SH; SO₃H; SO₃(C₁-C₆-alkyl); SO₂(C₁-C₆-alkyl); SO₂NH₂; SO₂NH(C₁-C₆-alkyl); SO₂NH (C₇-C₂₀-arylalkyl); SO(C₁-C₆-alkyl); or R₅ and R₆ together form a phenyl ring, which may be unsubstituted or substituted with halogen, nitro, cyano, thiocyanato; C₁-C₆-alkyl; trifluormethyl; C₁-C₆-alkoxy, CO₂H, CO(C₁-C₆-alky), amino, C₁-C₆-monoalkylamino, C₁-C₆-dialkylamino, SH; SO₃H; H; SO₃(C₁-C₆-alkyl), SO₂(C₁-C₆-alkyl), SO(C₁-C₆-alkyl), and
pharmacologically acceptable salts thereof.

By aryl the following definitions are intended throughout the whole patent application.

Aryl may be unsubstituted or mono-, di- or trisubstituted independently with hydroxy, halo, nitro, cyano, thiocyanato, trifluoromethyl, C₁-C₃ alkyl, C₁-C₃ alkoxy, CO₂H, CO₂ (C₁- C₃)alkyl, CONH₂, CONH(C₁-C₃ alkyl), CON(C₁-C₃ alkyl)₂, CO(C₁-C₃ alkyl), amino, (C₁-C₃ monoalkyl)amino, (C₁-C₃ dialkyl)amino, C₅-C₆ cycloalkylamino, (C₁-C₃ alkanoyl)amino, SH, SO₃H, SO₃ (C₁-C₃ alkyl), SO₂ (C₁-C₃ alkyl), SO(C₁-C₃ alkyl), C₁-C₃ alkylthio or C₁-C₃ alkanoylthio.

In all of the above mentioned substitution patterns it is preferred when R₁ to R₆ and X are selected as defined below, wherein these preferred definitions are preferred individually as well as in any conceivable combination, i.e. it is preferred that R₁ alone is as defined below, or that R₁ and R₂ are as defined below, or that R₁ and R₃ are as defined below, i.e. including all possible combinations:
R₁: alkenyl, arylalkenyl, cycloalkylalkyl, all as defined above;
R₂: alkoxy, arylalkyloxy, alkenyloxy, arylalkenyloxy, all as defined above;
R₃: alkyl, arylalkyl, alkenyl, all as defined above;
R₄: alkoxy, alkyloxyalkyloxy, alkenyloxy, all as defined above;
R₅ and R₆: independently selected from hydrogen, nitro, cyano, chloro, fluoro, bromo, trifluoromethyl; CO₂H; CO₂ CH₃, CONH₂; CONH CH₃, CH₃, SH; SO₂NH₂; N(CH₃)₂,
SO₂ CH₃;
X: O, NH, N CH₃, N-benzyl, N-allyl.

In a preferred embodiment
R₁ is selected from allyl, cinnamyl, cyclopropylmethyl or cyclobutylmethyl;
R₂ is selected from methoxy, ethoxy, n-propyloxy, benzyloxy, benzyloxy substituted in the aromatic ring with F, Cl, NO₂, CN, CF₃, CH₃ or OCH₃; allyloxy, cinnamyloxy or 3-phenylpropyloxy;
R₃ is selected from hydrogen, methyl, ethyl, benzyl or allyl;
R₄ is selected from hydroxy, methoxy, methoxymethoxy or acetyloxy;
R₅ and R₆ are each and independently selected from hydrogen, nitro, cyano, chloro,
fluoro, bromo, trifluoromethyl; CO₂H; CO₂ CH₃, CONH₂; CONH CH₃, CH₃, SH; SO₂NH₂; N(CH₃)₂, SO₂ CH₃; and
X is selected from O, NH, N CH₃, N-benzyl, N-allyl.

In an especially preferred embodiment R₁ is allyl or cyclopropylmethyl;
R₂ is selected from methoxy, ethoxy, n-propyloxy, benzyloxy substituted in the aromatic ring with chlorine;
R₃ is selected from hydrogen or CH₃;
R₄ is hydroxy.
R₅ and R₆ are each independently selected from hydrogen, CO₂H, CONH₂, SO₂NH₂ or SO₂CH₃; and
X is selected from O or NH.

The best mode known at present is to use the compounds of Examples 1, 6, 8, 18, 24, 41 and 42.

The novel compounds according to the invention are useful as agents for the treatment and/or prevention of disorders associated with the target calcineurin. The compounds as defined herein are in particular suitable for the preparation of a medicament for the treatment and/or prevention of glaucoma and/or uveitis.

Pharmaceutically and pharmacologically acceptable salts of the compounds of formula I are also comprised in the invention. Suitable salts are inorganic salts such as HCl salt, HBr salt, sulfuric acid salt, phosphoric acid salt. Organic acid salts such as methanesulfonic acid salt, salicylic acid salt, fumaric acid salt, maleic acid salt, succinic acid salt, aspartic acid salt, citric acid salt, oxalic acid salt, orotic acid salt, can also be used according to the invention.

The present invention is related to new possibilities for the therapeutic use of compounds known as δ-opioid receptor antagonists.
The new possibilities for the therapeutic use of δ-opiold receptor antagonists are related in part on the basis of the surprising identification of a second target - beside the target δ-oploid receptors - namely calcineurin. Calcineurin inhibition was proved with several of the δ-opioid receptor antagonists disclosed herein.
The present invention is related to the use of the δ-opioid receptor antagonists and their pharmaceutically acceptable salts as disclosed here for the prevention and/or treatment of glaucoma and/or uveitis.

Without being bound to any particular theory it is believed that the above mentioned indications can be treated with the compounds disclosed and claimed in view of the particular activity disclosed and shown later in the application and the therewith associated biochemical pathways.

In this connection the experimental results presented below show a further activity of the compounds defined herein. According to the test results an inhibition of the generation of interleukin-2 could be achieved with the compounds defined in the present invention, enabling the treatment and or prophylaxis of disorders requiring such an inhibition.

The compounds according to formula (I) or their pharmaceutically acceptable salts may be administered orally, intravenously, intraarterially, intramuscularly, intrathecally, intralumbarly, intraperitoneally, intranasally, intradermally, subcutaneously, epicutaneously, topically, transdermally, rectally, pulmonarily, conjunctivally, buccally, lingually or sublingually in a pharmaceutical formulation such as a solution, capsule, tablet, spray, suppository, ointment, cream, paste, plaster, patch.

The suitability of the compounds to be used in accordance with the present invention was evaluated using the following test methods.

### METHOD

### Calcineurin inhibition assays

Calcineurin inhibition assay was performed according to a described procedure (R. Baumgrass et al., J. Biol. Chem. 276, 47914, 2001).

Calcineurin inhibition was measured in a concentration range of 1-50 µM of the test compound at optimal Ca²⁺ and calmoduline concentrations. The calcineurin/compound mixtures were equilibrated in the assay buffer (40 mM Tris-HCl, pH 7.5, 100 mM NaCl, 6 mM MgCl₂, 0.5 mM ditiothreitol, 1 mM CaCl₂, 0.1 mg/ml bovine serum albumin) at 22°C for 30 min. Calcineurin activity was referenced to the assay lacking the test compounds. The data were computed with the SigmaPlot program.

### RESULTS

### Direct inhibition of calcineurin

**Inhibitory potency of calcineurin by δ-opioid receptor antagonists**

| **Compound** | **IC₅₀ (µM)** |
|---|---|
| HS 881 | 10 |
| HS 879 | 12 |
| HS 573 | 44 |
| HS 378 | 59 |
| Naltrindole | >100 |

**Inhibition of calcineurin mediated by δ-opioid receptor antagonists at a concentration of 20 µM**

| **Compound** | **Calcineurin activity (% of control)** |
|---|---|
| Naltrindole | 93 |
| HS 378 | 92 |
| HS 573 | 76 |
| HS 884 | 65 |
| HS 882 | 49 |
| HS 881 | 37 |
| HS 894 | 30 |
| HS 879 | 29 |

Unlike cyclosporin and FK506 (tacrolimus) which require prior binding to matchmaker proteins to inhibit calcineurin, the δ-opioid receptor antagonists produce a direct inhibition of the phosphatase activity of calcineurin.

Further studies were performed to confirm the calcineurin inhibitory action of the δ-opioid receptor antagonists. It is known that inhibition of calcineurin leads to an inhibition in the expression of interleukin-2.

### METHOD

### Measurement of interleukin-2 production in concanavalin A stimulated lymphocytes

Lymph nodes from collagen injected Dark-Agouti rats were used 10 days post-immunization to prepare individual single cell suspensions according to the described procedure (Kleinau et al., Journal of Autoimmunity 4, 871, 1991).
Cells were incubated with 5 µg/ml ConA (concanavaline A) and increasing concentrations of the δ-opioid receptor antagonists or cyclosporine A (0.01-10 µM). Cells were harvested 48 h after addition of the test compound and supernatants were collected and used for quantification of interleukin-2 by ELISA (Enyme-linked immunoabsorbant assay technique). Absorbance at 405 nm was measured. The data were computed with the GraphPad program. Results are expressed as inhibition percentage from the control, in the absence of the test compound.

### RESULTS

### Effect of δ-opioid receptor antagonists in interleukine-2 production in supernatants of ConA-stimulated lymphocytes

### Measurement of interleukin-2 production in MLR (Mixed Lymphocyte Reaction)

Measurement of interleukin-2 production in MLR was performed according to the described procedure (Gaveriaux-Ruff et al., J. Pharmacol. Exper. Ther. 298, 1193, 2001).
Splenocytes from mice were cultured with allogeneic stimulators (mitomycin treated BALB/c spleen cells). Mixed lymphocyte reaction was performed in the absence or presence of δ-opioid receptor antagonists and cyclosporin A. Interleukin-2 was measured in cell supernatant after 48 h of culture using ELISA.

### RESULTS

**Effect of opioid compounds on interleukin-2 (IL-2) production on spleen cells in the MLR**

| Naltrindole | HS 378 | Cyclosporin A | IL-2 |
|---|---|---|---|
| µM | µM | *nM* | *pg*/*ml* |
| | | | 84 ± 18 |
| 3 | | | 66 ± 18 |
| 10 | | | 29 ± 6 |
| 30 | | | 19 ± 5 |
| | 2 | | 49 ± 7 |
| | 10 | | 31 ± 3 |
| | 20 | | 22 ± 2 |
| | | 3 | 76 ± 27 |
| | | 10 | 41 ± 11 |
| | | 30 | 26 ± 2 |

Cyclosporin A suppressed interleukin-2 production. The δ-opioid receptor antagonists also were found to inhibit interleukin-2 production, indicating that such compounds suppress lymphocyte proliferation via an interleukin-2-dependent mechanism, thus directing towards a calcineurin pathway.

HS 378 is compound 1 as obtained in example 1
HS 573 is 17-(Cyclobutylmethyl)-6,7-didehydro-4,5α-epoxy-14β-ethoxy-5β-methylindolo[2',3':6,7]morphinan-3-ol Hydrochloride
HS 879 is 14β-(Benzyloxy)-17-(cyclopropylmethyl)-6,7-didehydro-4,5α-epoxyindolo[2',3':6,7]morphinan-3-ol
HS 881 is 14β-[(4-Chlorobenzyl)oxy]-17-(cyclopropylmethyl)-6,7-didehydro-4,5α-epoxyindolo[2',3':6,7]morphinan-3-ol Hydrochloride
HS 882 is 17-(Cyclopropylmethyl)-6,7-didehydro-4,5α-epoxy-14β-[(2-phenylbenzyl)oxy]indolo[2',3':6,7]morphinan-3-ol Hydrochloride
HS 884 is 14β-[(4-tert.-Butylbenzyl)oxy]-17-(cyclopropylmethyl)-6,7-didehydro-4,5α-epoxyindolo[2',3':6,7]morphinan-3-ol Hydrochloride
HS 894 is 17-(Cyclopropylmethyl)-6,7-didehydro-4,5a-epoxy-14β-[(3-phenylpropyl)oxy]indolo[2',3':6,7]morphinan-3-ol

### Preparation

The compounds represented by formula (I) may be obtained by the following methods:
Thebaine of the formula 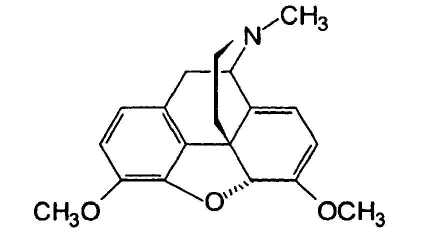 is being treated with dialkylsulfates, fluorosulfonic acid alkyl esters, alkylsulfonic acid alkyl esters, arylsulfonic acid alkylesters, alkyl halides, aralkyl halides, alkylsulfonic acid aralkyl esters, arylsulfonic acid aralkyl, arylalkenyl halides, chloroformates, in solvents such as tetrahydrofurane or diethyl ether using a strong base such as n-butyl lithium, lithium diethyl amide or lithium diisopropyl amide at low temperatures (-20 to - 80°C) (s. Boden et al., J.Org.Chem., Vol. 47: 1347-1349, 1982; Schmidhammer et al., Helv.Chim.Acta, Vol.71:642-647, 1988), giving compounds of the formula II 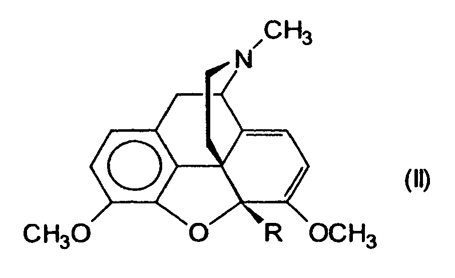
wherein
R is C₁-C₆ alkyl; C₁-C₆ alkenyl; C₇-C₁₆ aralkyl wherein the aryl is C₆-C₁₀ aryl and the alkyl is C₁-C₆ alkyl; C₇-C₁₆ arylalkenyl wherein the aryl is C₆-C₁₀ aryl and the alkenyl is C₁-C₆ alkenyl; alkoxyalkyl wherein the alkoxy is C₁-C₆ alkoxy and the alkyl is C₁-C₆ alkyl; CO₂ (C₁-C₆ alkyl); The substituted thebaine derivatives (formula (II)) or thebaine are converted into the corresponding 14-hydroxycodeinones according to formula III 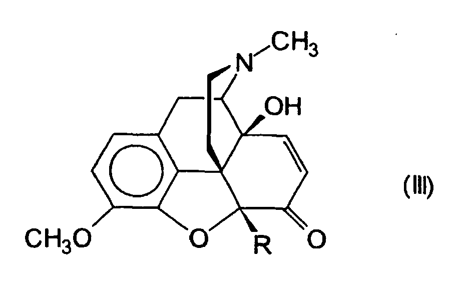 wherein
R is as defined above or being hydrogen,
by reaction with performic acid (s. Schmidhammer et al., Helv.Chim.Acta, Vol. 71:1801-1804, 1988) or m-chloroperbenzoic acid at a temperature between 0 and 60 °C. The preferred procedure is the reaction with performic acid at 0-10°C (H. Schmidhammer et al., Helv.Chim.Acta, Vol. 71:1801-1804,1988). These 14-hydroxycodeinones being treated with dialkyl sulfates, alkyl halides, alkenyl halides, aralkyl halides, arylalkenyl halides, chloroformates, in solvents such as N,N-dimethyl formamide or tetrahydrofurane using a strong base such as sodium hydride, potassium hydride or sodium amide giving compounds of formula (IV), 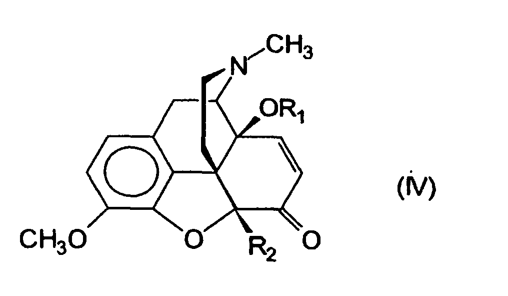 wherein
R₁ is C₁-C₆ alkyl, C₁-C₆ alkenyl, C₇-C₁₆ arylalkyl wherein the aryl is C₆-C₁₀ aryl and the alkyl is C₁-C₆ alkyl, C₇-C₁₆ arylalkenyl wherein the aryl is C₆-C₁₀ aryl and the alkenyl is C₁-C₆ alkenyl, C₁-C₆ alkanoyl, C₇-C₂₀ arylalkanoyl wherein the aryl is C₆-C₁₄ aryl and the alkyl is C₁-C₆ alkyl, C₇-C₂₀ arylalkenoyl wherein the aryl is C₆-C₁₄ aryl and the alkyl is C₁-C₆ alkenoyl;
R₂ is hydrogen; C₁-C₆ alkyl; C₁-C₆ alkenyl C₇-C₁₆ arylalkyl wherein the aryl is C₆-C₁₀ aryl and the alkyl is C₁-C₆ alkyl; C₇-C₁₆ arylalkenyl wherein the aryl is C₆-C₁₀ aryl and the alkenyl is C₁-C₆ alkenyl; alkoxyalkyl wherein the alkoxy is C₁-C₆ alkoxy and the alkyl is C₁-C₆ alkyl; CO₂(C₁-C₆ alkyl);
which in turn are reduced by catalytic hydrogenation using a catlayst such as palladium on charcoal and solvents such as methanol, ethanol or glacial acetic acid to give compounds of formula (V) 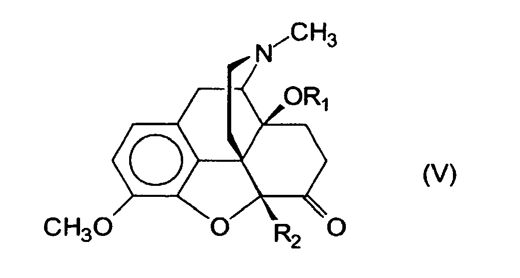 wherein
R₁ is C₁-C₆ alkyl, C₇-C₁₆ arylalkyl wherein the aryl is C₆-C₁₀ aryl and the alkyl is C₁-C₆ alkyl, C₁-C₆ alkanoyl, C₇-C₁₆ arylalkanoyl wherein the aryl is C₆-C₁₀ aryl and the alkanoyl is C₁-C₆ alkanoyl; and
R₂ is hydrogen; C₁-C₆ alkyl, C₁-C₆ alkenyl C₇-C₁₆ arylalkyl wherein the aryl C₆-C₁₀ aryl and the alkyl is C₁-C₆ alkyl; C₇-C₁₆ arylalkenyl wherein the aryl is C₆-C₁₀ aryl and alkenyl is C₁-C₆ alkenyl; alkoxyalkyl wherein the alkoxy is C₁-C₆ alkyl; CO₂(C₁-C₆ alkyl);

Thereafter N-demethylation can be carried out using chloroformates or cyanogen bromide to give intermediates of formula (VI) 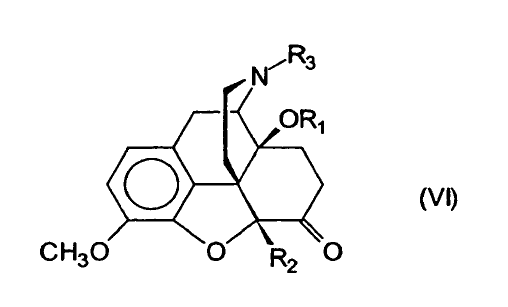 wherein
R₁ and R₂ are as defined above in formula (IV); and
R₃ is CO₂CHClCH₃, CO₂CH=CH₂, CO₂CH₂CCl₃, CO₂CH₂CH₃, CO₂Ph, CN or the like.

The intermediate carbamates of formula (VI) can be cleaved by refluxing in alcohols (in the case of 1-chloroethyl carbamates), by addition of hydrogen halides or halogen and subsequent refluxing in alcohols (in the case of vinyl carbamates), or by reductive cleavage using zinc in glacial acetic acid or methanol (in the case of 2,2,2-trichloroethyl carbamates). Other carbonates may be cleaved using aqueous acid, alkali or hydrazine. The intermediate cyanamides of formula (VI) can be cleaved by acid hydrolysis. Alkylation of the corresponding N-nor derivatives of formula (VII) 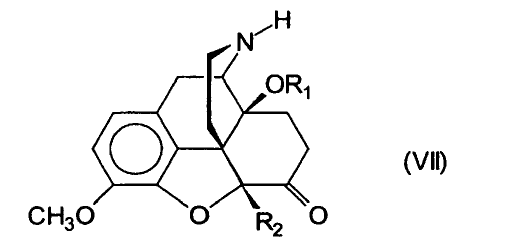 wherein
R₁ and R₂ are as defined above in formula (V), can be accomplished with alkenyl halides, cycloalkylalkyl halides, cycloalkenylalkyl halides, aralkyl halides, arylalkenyl halides, in solvents such as dichloromethane, chloroform, or N,N-dimethyl formamide in the presence of a base such as sodium hydrogen carbonate or potassium carbonate to yield derivatives of formula (VIII) 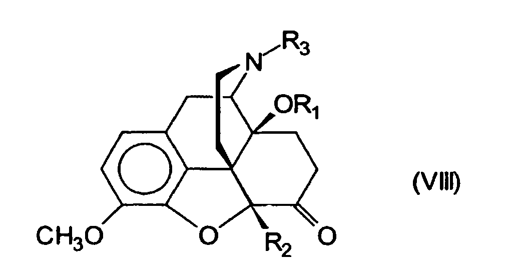 wherein
R₁ and R₂ are as defined above in formula (V); and
R₃ represents C₁-C₆ alkenyl; C₇-C₁₆ arylalkyl wherein the aryl is C₆-C₁₀ aryl and the alkyl is C₁-C₆ alkyl; C₇-C₁₆ arylalkenyl wherein the aryl is C₆-C₁₀ aryl and the alkenyl is C₁-C₆ alkenyl; C₄-C₁₀ cycloalkylalkyl wherein the cycloalkyl is C₃-C₆ cycloalkyl and the alkyl is C₁-C₄ alkyl; C₄-C₁₀ cycloalkylalkenyl wherein the cycloalkenyl is C₃-C₆ cycloalkenyl and the alkyl is C₁-C₄ alkyl;

Ether cleavage can be carried out using boron tribromide (in a solvent such as dichloromethane or chloroform at about 0 °C), 48 % hydrobromic acid (reflux), or other well known reagents for ether cleavage. The resulting phenols of formula (IX) 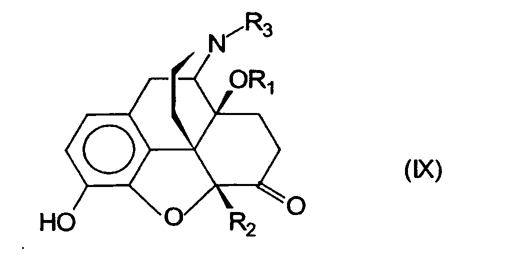 wherein
R₁, R₂ and R₃ are as defined above,
are being alkylated using alkyl halides, alkyl sulfates, sulfonic acid esters, aralkyl halides, arylalkenyl halides or acylated using carbonic acid chlorides, or carbonic acid esters to yield compounds of formula (X) 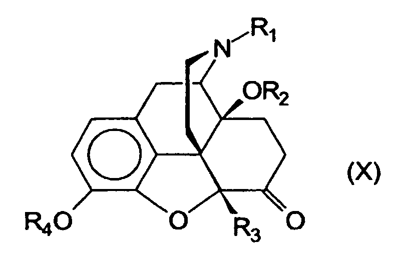 wherein
R₁, R₂ and R₃ are as defined above; and
R₄ is hydrogen, C₁-C₆ alkyl, C₇-C₁₆ arylalkyl wherein the aryl is C₆-C₁₀ aryl and the alkyl is C₁-C₆ alkyl, C₁-C₆ alkenyl, C₇-C₁₆ arylalkenyl wherein the aryl is C₆-C₁₀ aryl and the alkenyl is C₁-C₆ alkenyl; C₁-C₆ alkanoyl, C₇-C₁₆ arylalkanoyl wherein the aryl is C₆-C₁₀ aryl and the alkanoyl is C₁-C₆ alkanoyl, C₂-C₁₀ alkyloxyalkyl wherein alkyloxy is C₁-C₄ alkyloxy and alkyl is C₁-C₆ alkyl.

Compounds of the formula (I) wherein R₂ is hydroxy may be obtained from compounds of formula (III) wherein R is defined as above. These compounds may be reduced by catalytic hydrogenation using a catalyst such as palladium on charcoal and solvents such as methanol, ethanol, or glacial acetic acid to give compounds of the formula (V) wherein R₁ is hydrogen and R₂ is defined for R in formula (II).

The following reaction sequence and procedures leading to compounds of formulas (VI), (VII), (VIII), (IX) and (X) wherein the substituent in position 14 is hydroxy and the other substitutents are defined as above, is analogous to the reaction sequence and procedures described above. Further conversion to compounds of the formula (I) wherein R₂ is hydroxy is described below.

Compounds of the formula (I) wherein R₂ is hydrogen may be obtained from compounds of the formula (II) wherein R is as defined above or hydrogen. Catalytic hydrogenation followed by acid hydrolysis (s. Boden et al., J. Org. Chem. Vol. 47:1347-1349, 1982) may provide compounds of formula (XI) 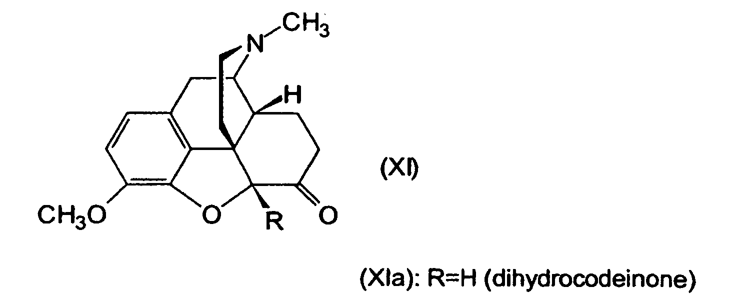 wherein R is as defined above in formula (II) or hydrogen.

Compounds of the formula (XI) and (XIa) (Mannich and Löwenheim, Arch.Pharm.Vol. 258:295, 1920) can be converted into compounds of formulas (V), (VI), (VII), (VIII), (IX), and (X) wherein the substituent in position 14 is hydrogen and R₂ and R₃ are as defined above, similarly as outlined above. Further conversion into compounds of the formula (I) wherein R₂ is hydrogen is described below.

Compounds of the formula (I) wherein R₄ is hydrogen may be prepared from compounds of the formula (IX) by alkylation with 5-chloro-1-phenyl-¹H-tetrazole to give the corresponding phenyltetrazolyl ethers of the formula XII) 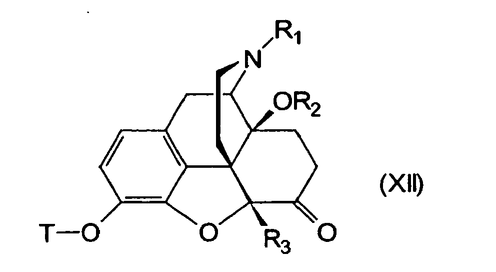 wherein R₁, R₂ and R₃ are as defined above and R₁ also can be CH₃, and T is phenyltetrazolyl.

Catalytic hydrogenation may afford (H. Schmidhammer et al., J. Med. Chem. Vol. 27:1575-1579, 1984) compounds of the formula (XIII) 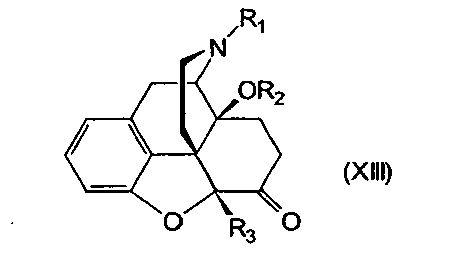 wherein R₁, R₂ and R₃ are as defined above and R₁ also can be CH₃;

In the case R₁ is CH₃, the N-methyl group has to be removed and the nitrogen alkylated as described above.

Alternatively, compounds of formula (I) wherein R₁ represents allyl or cyclopropylmethyl and R₃ represents H can be obtained by a shorter route starting either from naloxone (XIVa) or naltrexone (XIVa). 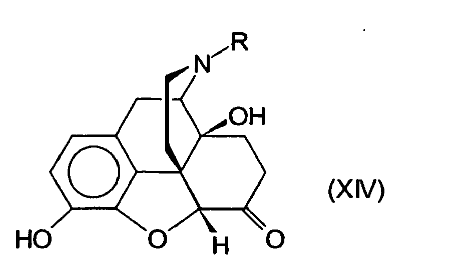 (XIVa): Naloxone - R is allyl
(XIVb): Naltrexone - R is cyclopropylmethyl.

The 3-hydroxy group of compounds of formula (XIV) is being protected by alkylation with benzyl bromide, methoxymethyl bromide, ethoxymethyl bromide or trityl chloride (triphenylmethyl chloride) in a solvent such as N,N-dimethyl formamide or dichloromethane in the presence of a base to yield compounds of formula (XV) 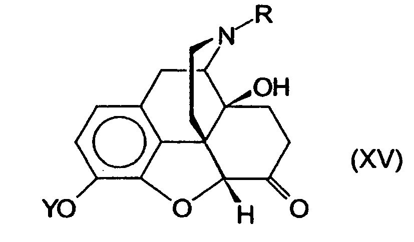 wherein
R is allyl or cyclopropylmethyl and Y = CH₂Ph, CH₂OCH₃, CH₂OC₂H₅ or C(Ph)₃.

These compounds are alkylated, alkenylated, cycloalkylalkylated, arylalkylated or arylalkenylated with dialkyl sulfates, alkyl halides, alkenyl halides, arylalkyl halides or arylalkenyl halides in solvents such as N,N-dimethyl formamide or tetrahydrofurane using a strong base such as sodium hydride, potassium hydride or sodium amide. The resulting 6-0,14-0-dialkylated compounds of formula (XVI) 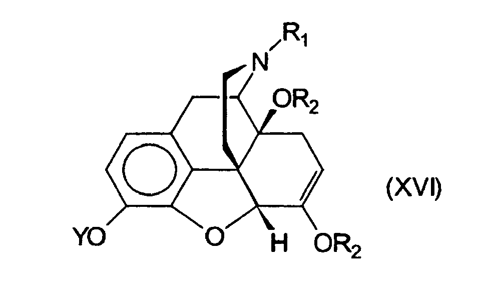 wherein
R₁ is allyl or cyclopropylmethyl; and
R₂ is C₁-C₆ alkyl, C₁-C₆ alkenyl, C₇-C₁₆ arylalkyl wherein the aryl is C₆-C₁₀ aryl and the alkyl is C₁-C₆ alkoxy, C₇-C₁₆ arylalkenyl wherein the aryl is C₆-C₁₀ aryl and alkenyl is C₁-C₆ alkenyl; and Y as defined above;
can be hydrolized with diluted acids like hydrochloric acid or sulfuric acid to afford compounds or formula (XVII) 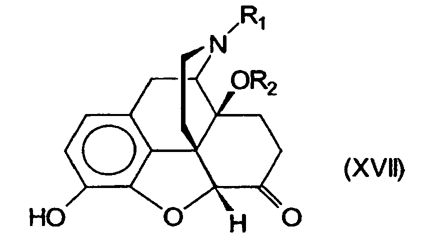 wherein
R₁ is allyl or cyclopropylmehtyl; and
R₂ is as defined above (formula XVI).

In the case R₂ is alkenyl or arylalkenyl the double bond may be reduced by catalytic hydrogenation to afford the corresponding saturated derivatives. Further conversion into compounds of formula (I) is described below.

Alternatively, compounds of formula (I) wherein R₁ represents allyl or cyclopropylmethyl and R₃ represents H can be prepared also via the following route:
The carbonyl group in position 6 of naloxone (XVa) and naltrexone (XVb), respectively, is being protected by reaction with ethylene glycol in the presence of an acid (e.g. methanesulfonic acid) at temperatures between 20 and 200 °C to give ketals of formula (XVIII) 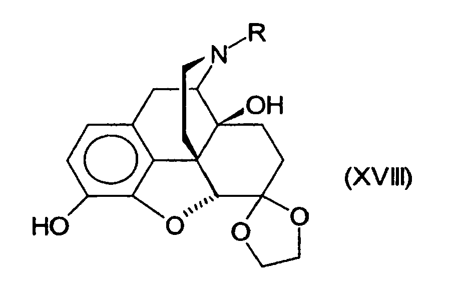
wherein R is allyl or cyclopropylmethyl.

The 3-hydroxy group of these ketals is being protected by alkylation with benzyl bromide, methoxymethyl bromide, ethoxymethyl bromide or trityl chloride in a solvent such as N,N-dimethyl formamide or dichloromethane in the presence of a base to yield compounds of formula (XIX) 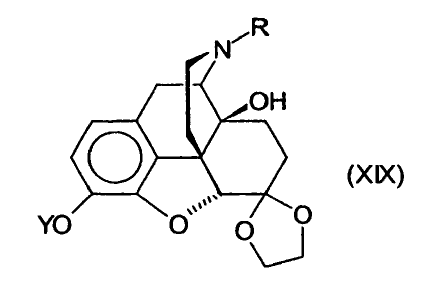 wherein R is allyl or cyclopropylmethyl and Y is as defined above.

These compounds are alkylated, alkenylated, arylalkylated or arylalkenylated with dialkyl suflates, alkyl halides, alkenyl halides, arylalkyl halides or arylalkenyl halides in solvents such as N,N-dimethyl formamide or tetrahydrofurane using a strong base such as sodium hydride, potassium hydride or sodium amide. The resulting compounds of formula (XX) 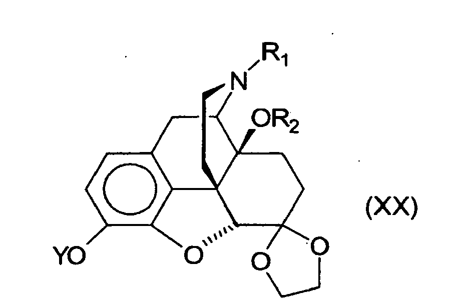 wherein R₁ is allyl or cyclopropylmethyl, R₂ is as defined above (formula (XVI)) and Y is as defined above
can be hydrolized in diluted acids like hydrochloride acid or sulfuric acid (a typical mixture for hydrolysis is: concentrated HCl: MeOH: H₂O 3/6/1 v/v/v) to afford compounds of formula (XVII). Compounds of formula (I) wherein R₁ represents allyl or cyclopropyl-ethyl, R₃ represents H, and X represents NH or O can be prepared from compounds of formula (XVII) as described below.

Compounds of the formula (I) wherein R₃ is as defined above and X represents NH are obtained by reaction of compounds of formula (VIII), (X) or (XIII) with phenylhydrazine or substituted phenylhydrazine in solvents such as methanol, ethanol or glacial acetic acid in the presence of methanesulfonic acid, HCl or HBr. Phenylhydrazine substituted at the aromatic ring with hydroxy, halogen, C₁-C₆ alkyl, C₁-C₆ alkoxy, amino, nitro, cyano, thiocyanato, trifluoromethyl, CO₂H, CO₂ (C₁-C₆) alkyl, CONH₂, CONH (C₁-C₆ alkyl), CON (C₁-C₆ alkyl)₂, SO₂NH₂, SO₂ (C₁-C₆) alkyl or the like may be employed. The reaction may be carried out at a temperature between 20 and 160 °C, preferably between 20 and 80 °C.

Compounds of formula (I) wherein R₃ is as defined above and X represents O are obtained by reaction of compounds of formula (VIII), (IX), (X) or (XIII) with O-phenylhydroxyl amine or substituted (at the aromatic ring) O-phenylhydroxyl-amine in solvents such as methanol ethanol, or glacial acetic acid in the presence of methanesulfonic acid, HCl or HBr. O-phenylhydroxylamine substituted at the aromatic ring with hydroxy, halogen, C₁-C₆ alkyl, C₁-C₆ alkoxy, amino, nitro, cyano, thiocyanato, trifluoromethyl, CO₂H, CO₂ (C₁-C₆) alkyl, CONH₂, CONH (C₁-C₆ alkyl), CON (C₁-C₆ alkyl)₂, SO₂NH₂, SO₂ (C₁-C₆) alkyl or the like may be employed.

The invention will now be described in more detail by the following examples.

### Examples

### Example 1

### Synthesis of 17-(Cyclopropylmethyl)-6,7-dehydro-4,5α-epoxy-14-ethoxy-3-hydroxy-5-methyl-6,7-2',3'-indolomorphinan hydrochloride (compound 1).

A mixture of 14-O-ethyl-5-methylnaltrexone (H. Schmidhammer et al, Helv. Chim. Acta, Vol. 76: 476-480, 1993) (580 mg, 1.51 mmol), phenylhydrazine hydrochloride (394 mg, 2.72 mmol), and 7 ml of glacial acetic acid was refluxed for 23 h. After cooling, the reaction mixture was poured on ice, alkalized with conc. NH₄OH and extracted with CH₂Cl₂ (3x30 ml). The combined organic layers were washed with H₂O (3x80 ml), dried over Na₂SO₄ and evaporated. The remaining residue (615 mg brownish foam) was dissolved in little MeOH and Et₂O/HCl was added. Thus, 550 mg (95%) of the compound 1 were isolated. For analysis a small amount was recrystallized from MeOH. m.p. > 260°C (dec.) IR (KBr): 3200 (⁺NH, NH, OH)cm⁻¹. Cl-MS:m/z 457 (M⁺ +1). ¹H-NMR ((d₆)DMSO): δ 11.34, 9.21. and 8.55 (3 s, ⁺NH, NH, OH), 7.32 (m, 2 arom. H), 7.08 (t, J = 8.1 Hz, 1 arom. H), 6.94 (t, J = 8.1 Hz, 1 arom. H), 6.62 (d, J = 8.1 Hz, 1 arom. H); 6.55 (d. J = 8.1 Hz, 1 arom. H), 1.86 (s, CH₃-C(5)), 1.01 (t, J = 6.8 Hz, 3H, CH₃CH₂O). Analysis calculated for C₂₉ H₃₂N₂O₃.HCLH₂O (511.06): C 68.16, H 6.90, N 5.48, Cl 6.94; found: C 67.87, H 6.88, N 5.30, Cl 7.28.

### Example 2

### Synthesis of 17-Allyl-6,7-dihydro-4,5α-epoxy-14-ethoxy-3-hydroxy-5-methyl-6,7-2',3'-indolomorphinan hydrochloride (compound 2).

A mixture of 14-O-ethyl-5-methylnaloxone (H. Schmidhammer et al., Helv. Chim. Acta Vol. 76:476-480, 1993) (1.2g, 2.66 mmol), phenylhydrazine hydrochloride (577 mg, 3.99 mmol), and 15 ml of glacial acetic acid was refluxed for 24 h. After cooling, the reaction mixture was poured on ice, alkalized with conc. NH₄OH and extracted with CH₂Cl₂ (3x80 ml), 1x30 ml). The combined organic layers were washed with H₂O (3x80 ml, 1x30 ml), dried over Na₂SO₄ and evaporated. The residue (1.3 g yellow-brown foam) was purified with column chromatography (alumina basic grade IV, elution with CH₂Cl₂). The corresponding fractions were combined and evaporated to give a colorless oil which was converted into the hydrochloride salt in the usual way and crystallized from MeOH/diethyl ether to yield 200 mg (17%) of the title compound 2. M.p. 168-170°C. IR (KBr):3200(⁺NH,OH)cm⁻¹. Cl-MS: M/z 443 (M⁺+1). ¹H-NMR (CD₃OD):
δ 7.39 (dd, J=7.8, 7.8 HZ, 2 arom. H), 7.14 (t, J=7.8 hz, 1 arom.H), 7.01 (t=7.8 HZ, 1 arom. H), 6.67 (s,2 arom. H), 6.02 (m, 1 olef. H), 5.72 (m, 2 olef. H), 1.99 (s, CH₃-C(5)), 1.09 (t, J=6.8 Hz, CH₃). Analysis calculated for C₂₈H₃₀N₂O₃. HCl. 1.5 H₂O (506.05): C 66.46, H 6.77 N 5.54, CI 7.01; found: C 66.55, 6.68, N 5.39, CI 6.98.

### Example 3

### Synthesis of 6,7-Dehydro-4,5α-epoxy-14-ethoxy-3-hydroxy-5-methyl-17-(2-phenyl)ethyl-6,7-2',3'-indolomorphinan hydrochloride (compound 5).

A mixture of 4,5α-epoxy-14-ethoxy-3-methoxy-5-methylmorphinan-6-one hydrochloride (H. Schmidhammer et al., Helv. Chim. Acta Vol. 76, 476-480,1993) (3.0 g, 7.88 mmol), potassium carbonate (3.9 g, 28.2 mmol), 2-phenylethyl bromide (1.41 ml, 10.4 mmol), and of 20 ml anhydrous N,N-dimethyl formamide was stirred at 80°C (bath temperature) for 2h. After cooling and addition of 130 ml of H₂O, the mixture was extracted with diethyl ether (3x60 ml). The combined organic layers were washed with H₂O (3x70 ml), dried over Na₂SO₄ and evaporated. The residue (3.6 yellow oil) was crystallized from MeOH to afford 2.1 g (70%) of 4,5α-epoxy-14-ethoxy-3-methoxy-5-methyl-17-(2-phenyl)ethylmorphinan-6-one (compound 3). M.p. 86-89°C. IR (KBr): 1725 (CO) cm-¹. CI-MS: m/z 448 (M⁺+1). ¹H-NMR (CDCl₃): δ 7.21 (m, 5 arom. H), 6.64 (d,J=8.2 Hz, 1 arom. H, 6.54 (d, J=8.2 Hz, 1 arom. H.), 3.85 (s, OCH₃), 1.60 (s, CH₃-C(5)), 1.12 (t, J=6.8 Hz, CH₃). Analysis calculated for C₂₈H₃₃NO₄ (447.55): C 75.14, H 7.43, N 3.13; found: C 75.04, H 7.69, N 3.26.

A solution of the compound 3 (1.5 g, 3.35 mmol) in 5 ml of 48% HBr was refluxed for 30 min and then evaporated. The residue was dissolved in MeOH and again evaporated (this procedure was repeated twice) to give a grey crystalline residue (1.7 g) which was treated with hot MeOH to yield 950 mg (63%) of the compound 4. M.p.>270°C. IR (KBr): 1720 (CO) cm⁻¹. CI-MS: m/z 434 (M⁺+1). ¹H-NMR (DMSO-d₆): δ 9.38 and 8.48 (2 s, ⁺NH, OH), 7.33 (m,5 arom. H), 6.68 (d, J=8.2 Hz, 1 arom. H), 6.64 (d, J=8.2 Hz, 1 arom. H), 1.51 (s, CH₃-C(5)), 1.34 (t, J=6.8 Hz, CH₃). Analysis calculated for C₂₇H₃₁NO₄. HBr (514.45):C 63.04, H 6.27, N 2.72, Br 15.53; found: C 63.15, H 6.48, N 2.61, Br 15.37.

A mixture of the compound 4 (700 mg, 1.61 mmol), phenylhydrazine hydrochloride (513 mg), 3.54 mmol), and 15 ml of glacial acetic acid was refluxed for 6 h. The reaction mixture was poured on ice, alkalized with conc. NH₄OH and extracted with CH₂Cl₂ (3x80 ml, 1x30 ml). The combined organic layers were washed with H₂O (3x80 ml), dried over Na₂SO₄ and evaporated. The residue (600 mg slightly brown foam) was converted into the hydrochloride salt in the usual way and crystallized from MeOH/diethyl ether to yield 360 mg (51%) of the title compound 5 as slightly pink crystals. M.p.>225°C. IR (KBr):3400 and 3200 (⁺NH, NH,OH). CI-MS:m/z 507 (M⁺+1). ¹H-NMR (DMSO-d₆): δ 11.34, 9.19 and 8.97 (⁺NH, NH, OH), 7.34 (m, 7 arom. H), 7.08 (t, J=7.9 Hz, 1 arom.), 6.94 (t, J=7,9 Hz, 1 arom. H), 6.62 (d, J=8.4 Hz, 1 arom. H), 6.57 (d, J=8.4 Hz, 1 arom. H), 1.87 (s, CH3-C(5)),0.96 (t, J=6.9 Hz, CH₃). Analysis calculated for C₃₃H₃₄N₂O₃. HCl.2 H₂O (579.14): C 68.44, H 6.79, N 4.84, Cl 6.12; found: C 68.81, H 6.55, N 4.72, Cl 6.40.

### Example 4

### Synthesis of 17-Allyl-6,7-dehydro-4,5α-epoxy-3-hydroxy-14-methoxy-5-methyl-6,7-2',3'-indolomorphinan hydrochloride (compound 6).

A mixture of 14-O-methyl-5-methylnaloxone (H. Schmidhammer et al., Helv. Chim. Acta Vol. 77:1585-1589, 1994) (1.0 g, 2.8 mmol), phenylhydrazine hydrochloride (728 mg, 5.04 mmol), and 15 ml of glacial acetic acid was refluxed for 24 h. After cooling, the reaction mixture was poured on ice, alkalized with conc. NH₄OH and extracted with CH₂Cl₂ (3x80 ml, 1x30 ml). The combined organic layers were washed with H₂O (3x80 ml), dried over Na₂SO₄ and evaporated. The residue (1.1 g brownish foam) was converted in the usual way into the hydrochloride salt and crystallized from acetone to yield 190 mg (19%) of the title compound 6 as slightly brown crystals. M.p. >280°C. IR (KBr): 3200 (⁺NH, NH,OH), ¹H-NMR: δ 7.32 (dd, J=7.9, 7.9 Hz, 2 atom. H), 7.06 (t, J=7.9 Hz, 1 arom. H), 6.93 (t, =7.9 Hz, 1 arom. H), 6,63 (d, J=8.2 Hz, 1 arom. H), 6.55 (d, J=8.2 Hz, 1 arom. H), 6.02 (m, 1olef.H), 5.63 (m, 1 olef. H), 3.15 (s, OCH₃), 2.07 (s, CH₃-C(5)). Analysis calculated for C₂₇H₂₈N₂O₃. HCl. 1.7 H₂O. 0.9 MeOH (524.44): C64.41, H 7.09, N 5.22; found: C 64.44, H 6.87, N 4.94.

### Example 5

### Synthesis of 6,7-Dehydro-4,5α-epoxy-3-hydroxy-14-methoxy-5-methyl-17-(2-phenyl)ethyl-6,7-2',3'-indolomorphinan Hydrochloride (compound 9).

A mixture of 4,5α-epoxy-3,14-dimethoxy-5-methylmorphinan-6-one hydrochloride (H. Schmidhammer et al., Helv. Chim. Acta Vol. 77:1585-1589, 1994) (2.24 g, 6.12 mmol), potassium carbonate (3.0 g, 21.9 mmol), 2-phenylethyl bromide (1.05 ml, 7.74 mmol), and 15 ml of anhydrous N,N-dimethyl formamide was stirred at 80°C (bath temperature) for 2 h. After cooling and addition of 110 ml of H₂O, the mixture was extracted with diethyl ether (3x60 ml). The combined organic layers were washed with H₂O (3x70 ml), dried over Na₂SO₄ and evaporated. The residue (2.9 yellow oil) was converted into the hydrobromide salt in the usual way and crystallized from MeOH to give 1.4 g (63%) of 4,5α-epoxy-3,14-dimethoxy-5-methyl-17-(2-phenyl)ethylmorphinan-6-one hydrobromide (compound 7) as colorless crystals. A small portion of this material was recrystallized from MeOH for analyses. M.p. 94-96°C. IR (KBr): 3400 (⁺NH), 1720 (CO) cm⁻¹. Cl-MS: m/z 434 (M⁺+1). ¹H-NMR (DMSO-d₆) δ 10.15 (s, ⁺NH), 7.30 (m, 5 arom. H), 6.74 (d, J=8.2 Hz, 1 arom. H), 6.68 (d, J=8.2 Hz, 1 arom.), 3.87 (s, OCH₃-C(3)), 3.58 (s, OCH₃-C(14)), 1.60 (s, CH₃-C(5)). Analysis calculated for C₂₇H₃₁NO₄. HBr (514.44): C 63.04, H 6.27, N 2.72; found: C 63.18, H 6.60, N 2.39.
A solution of the compound 7 (1.4 g, 3.32 mmol) in 5 ml of 48% HBr was refluxed for 30 min and then evaporated. The residue was dissolved in MeOH and again evaporated (this operation was repeated once) to afford a brownish crystalline residue (1.8 g) which was treated with hot MeOH to yield 590 mg (42%) of the compound 8.HBr. A small portion was recrystallized for analyses. M.p.>316°C. IR (KBr):3400 (⁺NH, OH), 1722 (CO)cm⁻¹. Cl-MS: m/z 420 (M⁺+1). ¹H-NMR (DMSO-d₆) δ 8.95 and 8.45 (2s, ⁺NH,OH), 6.90 (m, 5 arom. H), 6.23 (dd, J=8.2, 8.2 Hz, 2 arom. H), 2.97 s, OCH3), 1.08 (s, CH₃-C(5)). Analysis calculated for C₂₆H₂₉NO₄. HBr. 0.2 MeoH (506.85): C 62.09, H 6.13, N 2.76, Br 16.77; found: C 61.79, H 6.18, N 2.63, Br 16.12.

A mixture of the compound 8. HBr (468 mg, 0.93 mmol), phenylhyrazine hydrochloride (343 mg, 2.36 mmol), and 15 ml of glacial acetic was refluxed for 7 h. After cooling, the reaction mixture was poured on ice, alkalized with con. NH₄OH and extracted with CH₂Cl₂ (3x70 ml, 1x30 ml). The combined organic layers were washed with H₂O (3x80 ml), dried over Na₂SO₄ and evaporated. The residue (410 mg slightly brown foam) was converted into the hydrochloride salt in the usual way and crystallized from MeOH/diethyl ether to give 390 mg (83%) of the title compound 9 as slightly pink crystals. An analytic sample was obtained by recrystallization of a small portion of this material from MeOH/diethyl ether. M.p. 257-260°C (dec.). IR (KBr): 3460 (⁺NH, NH, OH) cm⁻¹. Cl-MS: m/z 493 (M⁺+1). ¹H-NMR (DMSO-d₆) δ 11.30, 9.20 and 9.05 (3 S, ⁺NH, NH, OH), 7.25 (m, 7 arom. H), 7.10 (t, J=8.2 Hz, 1 arom. H), 6.96 (t, J=8.2 Hz, 1 arom. H), 6.59 (dd, J= 8.2, 8.2 Hz, 2 arom. H), 3.32 (s, OCH₃), 1.87 (s, CH₃-C(5)). Analysis calculated for C₃₂H₃₂N₂O₃. HCl. 3.7 MeOH (647.63): C 66.21, H 7.44, N 4.33; found: C 66.04, H 7.13, N 4.60.

### Example 6

### Synthesis of 17-(Cyclopropylmethyl)-6,7-dehydro-4,5a-epoxy-3-hydroxy-14-methoxy-5-methyl-6,7-2',3'indolomorphinan Hydrochloride (compound 10).

A mixture of 14-O-methyl-5-methylnaltrexone (H.Schmidhammer et al., Helv. Chim. Acta Vol. 77: 1585-1589, 1994) (620 mg, 1.68 mmol), phenylhydrazine hydrochloride (365 mg, 2.52 mmol), and 7 ml of glacial acetic acid was refluxed for 17.5 h. After cooling, the reaction mixture was poured on ice, alkalized with NH₄OH and extracted with CH₂Cl₂ (3x70 ml, 1x20 ml). The combined organic layers were washed with H₂O (3x80 ml), dried over Na₂SO₄ and evaporated. The residue (1.11 g brown foam) was purified by column chromatography (silica gel 230-400 mesh, mobile phase CH₂Cl₂/MeOH 90:9). The corresponding fractions were combined and evaporated to afford a slightly yellow foam which was dissolved in MeOH and treated with ethereal HCl to yield 520 mg (65%) of the compound 10 as colorless crystals. For analyses a small sample was recrystallized from MeOH. M.p. >250°C (dec.). IR (KBr):3515 and 3220 (⁺NH, NH, OH)cm⁻¹. Cl-MS: m/z 443 (M⁺+1). ¹H-NMR (DMSO-d₆): δ 11.30, 9.12, 8.93 (3 s,⁺NH, NH, OH), 7.34 (m, 2 arom. H), 7.09 (t, J=8.3 Hz, 1 arom. H), 6.95 (t, J=8.3 HZ, 1 arom. H), 6.63 (d, J=8.1 Hz, 1 arom. H), 6.56 (d, J=8.1 Hz, 1 arom. H), 3.24 (s, OCH₃), 1.87 (s, CH₃-C(5)). Analysis calculated for C₂₈H₃₀N₂O₃. HCl. 0.7 H₂O (491.67):C 68.41, H 6.64, N 5.70, Cl 7.21; found: C 68.52, H 6.86, N 5.65, Cl 7.48.

### Example 7

### Synthesis of 17-Allyl-6,7-dehydro-4,5a-epoxy-3-hydroxy-5-methyl-14-n-propyloxy-6,7-2',3'-indolomorphinan. CH₃SO₃H (compound 15).

A mixture of 7,8-dihydro-5-methyl-14-n-propyloxycodeinone (9; 2.67 g, 7.19 mmol), KHCO₃ (3.6 g, 35.93 mmol), 1-chloroethyl chloroformate (4.73 ml, 43.12 mmol), and 35 ml of 1,2-dichloroethane was stirred under reflux for 3.5 h. After cooling, the inorganic material was filtered off and the filtrate evaporated. The residue (4.67 g of a yellowish oil of 17-(1-chloroethoxy)- carbonyl-4,5α-epoxy-3-methoxy-5-methyl-14-n-propyloxymorphinan-6-one (compound 11); pure by TLC) was not further purified and
characterized. A solution of the compound 11 in MeOH was refluxed for 1 h and then evaporated. The residue (3.54 g slightly brown foam) was crystallized from 2.5 ml MeOH/2 ml diethyl ether to give 1.68 g (66%) of 4,5α-epoxy-3-methoxy-5-methyl-14-n-propyloxy-morphinan-6-one hydrochloride (compound 12). M.p. 186-188°C. IR (KBr): 3425 (⁺NH₂), 1725 (CO)cm⁻¹. EI-MS: m/z 357 (M⁺). ¹H-NMR (DMSO-d₆): σ 10.11 and 8.15 (2 broad s, ⁺NH₂), 6.83 (d, J=8.2 Hz, 1 arom. H), 6.74 (d, J=8.2 Hz, 1 arom. H), 3.78 (s, CH₃O), 1.48 (s, CH₃-C(5)), 0.95 (t, J=7.4 Hz, CH₃). Analysis calculated for C₂₁H₂₇NO₄. HCl. 0.6 MeOH (413.14): C 62.80, H 7.42, N 3.39, Cl 8.58; found: C 62.66, H 7.34, N 3.40, Cl 8.98. A mixture of the compound 12 (1.45 g, 3.68 mmol), allyl bromide (0.36 ml, 4.06 mmol), potassium carbonate (2.9 g, 20.8 mmol), and 10 ml of anhydrous N,N-dimethyl formamide was stirred at 80°C (bath temperature) for 1.5 h. The inorganic solid was filtered off and the filtrate evaporated to give 1.7 g of a yellowish oily residue. This residue was partitioned between CH₂Cl₂ and H₂O. The organic layer was washed with H₂O and brine, dried over Na₂SO₄ and evaporated. The residue (1.375 g of a slightly yellow oil) was crystallized from ethanol to yield 1.28 g (88%) of 17-allyl-4,5α-epoxy-3-methoxy-5-methyl-14-n-propyloxymorphinan-6-one (compound 13) as slightly yellow crystals. M.p. 122-124°C. IR(KBr): 1720 (CO)cm⁻¹. EI-MS: m/z 397 (M⁺). ¹H-NMR (CDCl₃): δ 6.63 (d, J=8.3 Hz, 1 arom. H), 6.55 (d, J=8.3 Hz, 1 arom. H), 5.79 (m, 1 olef. H), 5.13 (m, 2 olef. H), 3.84 (s, OCH3), 1.60 (s, CH₃-C(5)), 1.00 (t, J=7.4 Hz, CH3). Analysis calculated for C₂₄H₃₁NO₄ (397.51): C 72.52, H 7.86, N 3.52; found: C 72.14, H 7.76, N 3.44. A 1 M solution of boron tribromide in CH₂Cl₂ (10.8 ml) was added to an ice-cooled solution of the compound 13 (577 mg, 1.45 mmol) in 75 ml of CH₂Cl₂ at once. After stirring at 0-5°C for 2 h, a mixture of 20 g ice and 4 ml of conc. NH₄OH was added. The resulting mixture was stirred at room temperature for 30 min and the extracted with CH₂Cl₂ (3x50 ml). The combined organic layers were washed with brine (70 ml), dried over Na₂SO₄ and evaporated. The residue (600 mg brownish foam) was converted into the hydrobromide salt in the usual way and crystallized from MeOH to afford 314 mg (47%) of 17-allyl-4,5α-epoxy-3-hydroxy-5-methyl-14-n-propyloxymorphinan-6-one hydrobromide (compound 14). M.p. 244-247°C (dec.). IR (KBr): 3441 and 3332 (⁺NH, OH), 6.68 (d, J=8.2 Hz, 1 arom. H), 6.62 (d, J=8.2 Hz, 1 arom. H), 5.92 (m, 1 olef. H), 5.67 (m,2 olef. H), 1.49 (s, CH₃-C(5)), 0.96 (t, J=7.2 Hz, CH₃).

A mixture of the compound 14 (300 mg, 0.65 mmol), phenylhydrazine hydrochloride (187 mg, 1.29 mmol), and 30 ml of glacial acetic acid was refluxed for 7.5 h. After cooling, the reaction mixture was poured on ice, alkalized with conc. NH₄OH and extracted with CH₂Cl₂ (3x60 ml). The combined organic layers were washed with H₂O (3x80 ml) and brine (50 ml), dried over Na₂SO₄ and evaporated. The residue (325 mg brownish foam) was converted into the methane sulfonate in the usual way and recrystallized from MeOH/diethyl ether to yield 264 mg (74%) of the title compound 15. Recrystallization of a small portion of this material from ethanol afforded an analytical sample. M.p. >256°C. FAB-MS: m/z 457 (M⁺+1), ¹H-NMR (DMSO-d₆): δ 11.29, 9.17 and 8.45 (3 s, ⁺NH, NH, OH), 7.32 (d, J=8.2 Hz, 2 arom. H), 7.10 (t, J=8.2 Hz, 1 arom. H), 6.94 (t, J=8.2 Hz, 1 arom. H), 6.59 (s, 2 arom. H), 5.90 (m, 1 olef. H), 5.68 (m, 2 olef. H), 1.88 (s, CH₃-C(5)), 0,55 (t, J=7.3 Hz, CH₃). Analysis calculated for C₂₉H₃₂N₂O₃H. 0.5 H₂O (561.70): C 64.15, H 66.4, N 4.99, S 5.72; found: C 64.08, H 6.87, N 5.09, S 5.87.

### Example 8

### Synthesis of 17-(Cyclopropylmethyl)-6,7-dehydro-4,5α-epoxy-3-hydroxy-5-methyl-14-n-propyloxy-6,7-2,3'-indolomorphinan. CH₃SO₃H (compound 18).

A mixture of 4,5α-epoxy-3-methoxy-5-methyl-14-n-propyloxymorphinan-6-one hydrochloride (compound 12 of Example 7) (1.46 g, 3.71 mmol), potassium carbonate (2.24 g, 16.24 mmol), cyclopropylmethyl chloride (0.43 ml, 4.44 mmol), and 15 ml of anhydrous N,N-dimethyl formamide was stirred at 85°C (bath temperature) for 36 h.

The inorganic solid was filtered off and the filtrate evaporated. A solution of the residue in 30 ml of CH₂Cl₂ was washed with H₂O (3x30 ml), dried over Na₂SO₄ and evaporated. The residue (1,69 g orange-yellow oil) was dissolved in diethyl ether and treated with ethereal HCl to give 920 mg (55%) of 17-(cyclopropylmethyl)-4,5α-epoxy-3-methoxy-5-methyl-14-n-propyloxymorphinan-6-one hydrochloride (compound 16) as colorless powder. M.p. 156-158°C. IR (KBr): 3400 (⁺NH), 1723 (CO) cm⁻¹. Cl-MS: m/z 412 (M⁺+1). ¹H-NMR (DMSO-d₆): δ 8.57 (s, ⁺NH), 6,85 (d, J=8.2 Hz, 1 arom. H), 6.75 (d, J=8.2 Hz, 1 arom. H), 3.79 (s, OCH₃), 1.51 (s, CH₃-C(5)), 0.97 (t, J=7.4 Hz, CH₃). Analysis calculated for C₂ₛH₃₃NO₄. HCl. 0.6 H₂O (458.81): C 65.45, H 7.73, N 3.05, Cl 7.73; found: C 65.45, H 7.85, N 3.08, Cl 7.84.

A 1 M solution of boron tribromide in CH₂Cl₂ (7.3 ml) was added at once to an ice-cooled solution of the compound 16 (480 mg, 0.97 mmol) in 50 ml of CH₂Cl₂. After 50 min stirring at 0-5°C, a mixture of 13 g ice and 3 ml conc. NH₄OH was added. The resulting mixture was stirred at room temperature for 30 min and the extracted with CH₂Cl₂ (3x30 ml). The combined organic layers were washed with brine (45 ml), dried over Na₂SO₄ and evaporated. The residue (204 mg slightly brown foam) was treated with 0.5 ml hot MeOH to afford 302 mg (55%) of 17-(cydopropylmethyl)-4,5α-epoxy-3-hydroxy-5-methyl-14-n-propyloxymorphinan-6-one (compound 17). M.p. 184-186°C. IR (KBr): 3390 (OH), 1720 (CO)cm ⁻¹. Cl-MS: m/z 397 (M⁺+1). ¹H-NMR (CDCl₃): δ 10.24 (broad s, OH), 6.73 (d, J=8.2 Hz, 1 arom. H), 6.65 (d, J=8.2 Hz, 1 arom. H)1.62 (s, CH₃-C(5)), 1.00 (t,J=7.3 Hz, CH3). Analysis calculated for C₂₄H₃₁NO₄. 0.6 MeOH (416.74): C 70.90, H 8.08, N 3.36; found: C 70.76, H 7.73, N 3.52.

A mixture of compound 17 (230 mg, 0.58 mmol), phenylhydrazine hydrochloride (142 mg, 0.98 mmol), and 23 ml of glacial acetic acid was refluxed for 3.5 h. After cooling, the reaction mixture was poured on ice, alkalized with con. NH₄OH and extracted with CH₂Cl₂ (3x40 ml). The combined organic layers were washed with H₂O 2x50 ml) and brine (50 ml), dried and evaporated. The residue (262 mg yellow-brown foam) was converted in the usual way into the methane sulfonate and crystallized from MeOH/diethyl ether to yield 204 mg (62%) of the compound 18. M.p. 295-298°C (dec.) FAB-MS: m/z 471 (M⁺+1). ¹H-NMR (DMSO-d₆) δ 11.27, 9.12 and 8.46 (3s, ⁺NH, NH, OH), 7.14 (m, 4 arom. H), 6.59 (s, 2 arom. H), 1.90 (s, CH3-C(5)), 0.67 (t, J=7.3 Hz, CH₃) Analysis calculated for C₃₀H₃₄N₂O₃. CH₃SO₃H. 1.5 H₂O (584.74): C 62.71, H 6.96, N 4.72, S 5.40; found: C 62.67, H 6.96, N 4.79, S 5.40.

Examples 9-24, and 28-30 illustrate further compounds, which can be prepared according to one of the methods described above.

### Example 9

### 17-(Cyclopropylmethyl)-6,7-dehydro-4,5α-epoxy-14-hydroxy-3-(methoxymethoxy)-6,7-2',3'-benzo[b]furanomorphinan (compound 19).

M.p.129-130°C. 1H-NMR (CDCl₃): δ 7.45 (d, J = 8.3 Hz, 1 arom. H), 7,37 (d, J = 8.3 Hz, 1 arom. H), 7.25 (m, 1 arom. H), 7.16 (m, 1 arom.), 6.86 (d, J = 8.3 Hz, 1 arom. H), 6.60 (d, J = 8.3 Hz, 1 arom. H), 5.63 (s, H-C(5)), 5.17 and 5.06 (2 d, J = 6.6, 6.6 Hz, OCH₂O), 3.42 (s, CH₃O).

### Example 10

### 17-Cyclopropylmethyl-6,7-dehydro-4,5α-epoxy-14-hydroxy-3-(methoxymethoxy)-6,7-2',3'-(N-methoxymethylindolo)morphinan (compound 20).

¹H NMR (CDCl₃): δ 7.44 (m, 2 arom. H), 7.20 (m, 1 arom. H), 7.07 (m, 1 arom. H), 6.82 (d,J = 8 Hz, 1 arom. H), 6.58 (J = 8 Hz), 5.81 (s, H-C(5)), 5.79 and 5.50 (2 d, J = 10.8, 10.8 Hz, NCH₂O), 5.12 and 5.50 (2 d, J = 6.4, 6.4 Hz, OCH₂O), 3,41 and 3.33 (2 s, 2 CH₃O).

### Example 11

### 17-(Cyclopropylmethyl)-6,7-dehydro-14-(2',6'-dichlorobenzyloxy)-4,5α-epoxy-14-3-(methoxymethoxy)-6,7-2',3'-benzo[b]furanomorphinan (compound 21).

M.p. 180-182 °C. 1 H NMR (CDCl₃): δ 7.41 (d, J = 8.3 Hz, 1 arom. H), 7.33 (d, J = 8.3 Hz, 1 arom. H), 7.23 (m, 1 arom. H) 7.14 (m, 2 arom. H), 7.03 and 7.01 (2 d, J = 7.3, 7.3 Hz), 6.84 (d, J, 8.3 Hz, 1 arom. H) 6.59 (d, J = 8.3 Hz, 1 arom. H), 5.56 (s, H-C(5)), 5.32 and 4.68 (2 d, J = 8.7, 8.7 Hz, OCH₂Ar), 5.16 and 5.05 (2 d, J = 6.6, 6.6 Hz, OCH₂O), 3.41 (s, CH₃O).

### Example 12

### 17-(Cyclopropylmethyl)-6,7-dehydro-14-(2',6'-dichlorobenzyloxy)-4,5α-epoxy-3-hydroxy-6,7-2',3'-benzo[b]furanomorphinan (compound 22).

M.p. 193-195 °C (dec). 1 H NMR (CDCl₃): δ 7.42 (d, J = 8.3 Hz, 1 arom. H), 7.33 (d, J = 8 Hz, 1 arom. H), 7.24 (m, 1 arom. H) 7.14 (m, 2 arom. H), 7.03 and 7.01 (2 d, J = 7.3 Hz, 1 arom. H), 6.64 (d, J, 8.1 Hz, 1 arom. H) 6.56 (d, J = 8.1 Hz, 1 arom. H), 5.58 (s, H-C(5)), 5.32 and 4.68 (2 d, J = 8.6 Hz, OCH₂ Ar).

### Example 13

### 17-(Cyclopropylmethyl)-6,7-dehydro-4,5α-epoxy-3-(methoxymethoxy)-14-(3'-nitrobenzyloxy)-6,7-2',3'-benzo[b]furanomorphinan (compound 23).

¹H NMR (CDCl₃): δ 8.25 (s, 1 arom. H), 7.28 (m, 4 arom. H), 7.15 (m, 1 arom. H) 6.87 (d, J = 8.3 Hz, 1 arom. H), 6.62 (d, J = 8.3 Hz, 1 arom. H), 5.66 (s, H-C(5)), 5.17 and 5.07 (2 d, J =6.6 Hz, OCH₂O) 4.92 and 4.44 (2 d, J = 11.5 Hz, OCH₂Ar), 3.42 (s, CH₃O).

### Example 14

### 17-(Cyclopropylmethyl)-6,7-dehydro-4,5a-epoxy-3-hydroxy-14-(3'-nitrobenzyloxy)-6,7-2',3'-benzo[b]furanomorphinan hydrochloride (compound 24).

M.p. > 230 °C (dec). 1 H NMR (DMCO-d6): δ 9.40 (s, OH), 9.15 (broad s, ⁺NH), 7.84 (s, 1 arom. H) 7.60 (d, J = 8.8 Hz, 1 arom. H), 7.53 (d, J = 7.6 Hz, 1 arom. H), 7.45 (d, J = 8 Hz, 1 arom. H) 7.23 (d, J = 7.6 Hz, 1 arom. H), 7.19 (d, J = 7.6 Hz, 1 arom. H), 6.98 (m, 1 arom. H) 6.88 (d, J = 7.6 Hz, 1 arom. H) 6.69 (d, J =8.3 Hz, 1 arom. H), 6.66 (d, J = 8.3 Hz, 1 arom. H), 6.03 (s, H-C(5)), 4.98 and 4.87 (2 d, J = 14, 14 Hz, OCH₂Ph).

### Example 15

### 17-(Cyclopropylmethyl)-6,7-dehydro-4,5α-epoxy-3-(methoxymethoxy)-14-(2-naphtylmethoxy)-6,7-2'-3'-benzo[b]furanmorphinan (compound 25).

M.p. 198-201 °C. 1 H NMR (CDCl₃): δ 7.72-7.08 (m, 11 arom. H), 6.86 (d, J = 8.3 Hz, 1 arom. H), 6.62 (d, J = 8.3 Hz, 1 arom. H), 5.68 (s, H-C(5)), 5.17 and 5.07 (2 d, J = 6.6, 6.6 Hz, OCH₂O), 5.01 and 4.57 (2 d, J = 11.2, 11.2 Hz, OCH₂Ar), 3,42 (s, CH₃O).

### Example 16

### 17-(Cyclopropylmethyl)-6,7-dehydro-4,5α-epoxy-3-hydroxy-14-(2'-naphtylmethoxy)-6,7-2',3'-benzo[b]furanomorphinan hydrochloride (compound 26).

M.p. > 215 °C. 1 H NMR (DMSO-d6): δ 9.42 (s, OH), 9.00 (broad s, ⁺NH), 7.68-6.85 (m, 11 arom. H), 6.71 (d, J = 8 Hz, 1 arom. H), 6.67 (d, J = 8 Hz, 1 arom. H), 6.04 (s, H-C(5)), 4.92 (s, OCH₂Ar).

### Example 17

### 17-(Cyclopropylmethyl)-6,7-dehydro-4,5α-epoxy-14-(2'-fluorobenzyloxy)-3-(methoxymethoxy)-6,7-2'-3'-benzo[b]furanmorphinan (compound 27).

¹H NMR (DMSO-d6): δ 7.56 (d, J = 8 Hz, 1 arom. H), 7.49 (d, J = 8 Hz, 1 arom. H), 7.31 (m, 1 arom. H), 7.21 (m, 1 arom . H), 6.81 (d, J = 8.4 Hz, 1 arom. H), 6.67 (d, J = 8.4 Hz), 5.72 (s, H-C(5)), 5.06 and 5.01 (2 d, J = 6.4, 6.4 Hz, OCH₂O), 4.89 and 4.57 (2 d, J = 11.6, 11.6 Hz, OCH₂ Ar), 3,33 (s, CH₃O).

### Example 18

### 17-(Cyclopropylmethyl)-6,7-dehydro-4,5α-epoxy-14-(2'-fluoro-benzyloxy)-3-hydroxy-6,7-2',3'-benzo[b]furanomorphinan Hydrochloride (compound 28).

M.p. > 215 °C. 1 H NMR (CDCl₃): δ 9.45 (s, OH), 9.04 (broad s, ⁺NH), 7.54 (d, J = 8.4 Hz, 1 arom. H) 7.31-6.73 (m, 7 arom. H), 6.71 (d, J = 8.2 Hz, 1 arom. H), 6.66 (d, J = 8.2 Hz, 1 arom. H), 5.98 (s, H-C(5)), 4.81 and 4.84 (2 d, J = 12 Hz, OCH₂ Ar).

### Example 19

### 14-Cinnamyloxy-17-(cyclopropylmethyl)-6,7-dehydro-4,5α-epoxy-3-(methoxymethoxy)-6,7-2'-3'-benzo[b]furanomorphinan (compound 29).

M.p. 156-159 °C. 1 H NMR (CDCl₃): δ 7.47 (d, J = 8 Hz, 1 arom. H), 7.33 (d, J = 8 Hz, 1 arom. H), 7.28-7.07 (m, 7 arom. H), 6.84 (d, J = 8.4 Hz, 1 arom. H), 6.59 (d, J = 8.4 Hz, 1 arom . H), 6.38 (d, J = 16 Hz, 1 olef. H), 6.13 (m, 1 olef. H), 5.68 (s, H-C(5)), 5.16 and 5.06 (2 d, J = 6.4, 6.4 Hz, OCH₂O), 4.46 and 4.11 (2 m,OCH₂Ar), 3,42 (s, CH₃O).

### Example 20

### 14-Cinnamyloxy-17-cyclopropylmethyl-6,7-dehydro-4,5α-epoxy-3-hydroxy-6,7-2'-3'-benzo[b]furanomorphinan Salicylate (compound 30).

¹H NMR (CDCl₃): δ 7.94 (d, J = 8 Hz, 1 arom. H), 7.35 (d, J = 8 Hz, 1 arom. H), 7.30-6.73 (m, 12 arom. H), 6.56 (d, J = 8 Hz, 1 arom. H), 5.96 (s, 2 olef. H), 5.55 (s, H-C(5)), 4.33-4.02 (m, OCH₂Ar).

### Example 21

### 17-(Cyclopropylmethyl)-6,7-dehydro-4,5α-epoxy-14-methoxy-3-(methoxymethoxy)-6,7-2'-3'-benzo[b]furanomorphinan (compound 31).

¹H NMR (DMSO-d6): δ 7.7.56 (d, J = 8 Hz, 1 arom. H), 7.52 (d, J = 8 Hz, 1 arom. H), 7.32 (dd, J = 8,8 Hz, 1 arom. H), 5.64 (s, H-C(5)), 5.05 and 5.00 (2 d, J = 6.4, 6.4 Hz, OCH₂O), 3.32 (CH₃O).

### Example 22

### 17-(Cyclopropylmethyl)-6,7-dehydro-4,5α-epoxy-3-hydroxy-14-methoxy-6,7-2'-3'-benzo[b]furanomorphinan hydrochloride (compound 32).

M.p. > 240 °C. ¹H NMR (DMSO-d6): δ 9.47 (s, OH), 9.17 (broad s, ⁺NH), 7.61 (d, J = 8 Hz, 1 arom. H), 7.53 (d, J = 8 Hz, 1 arom. H), 7.36 (dd, J = 8, 8 Hz, 1 arom. H), 7.27 (dd, J = 8, 8 Hz, 1 arom. H), 6.72 (d, J = 8.4 Hz, 1 arom. H), 6.65 (d, J = 8.4 Hz, 1 arom. H), 5.90 (s, H-C(5)), 3.35 (s, CH₃O).

### Example 23

### 17-(Cyclopropylmethyl)-14-(2'-chlorobenzyloxy)-6,7-dehydro-4,5α-epoxy-3-(methoxymethoxy)-6,7-2'-3'-(N-methoxymethylindolo)morphinan (compound 33).

¹H NMR (CDCl₃): δ 7.56 (m, 1 arom. H), 7.44 (m, 1 arom. H), 7.37-7.17 (m, 3 arom H), 7.01 (m, 1 arom. H), 6.91 (m, 1 arom. H), 6.83 (d, J = 8.2 Hz, 1 arom. H), 6.59 (dd, J = 8.2, 8.2 Hz, 1 arom. H), 5.90 (s, H-C(5)), 5.82 and 5.55 (2 d, J = 11.2, 11.2 Hz, NCH₂O), 5.13 and 5.03 (2 d, J = 6.4, 6.4 Hz, OCH₂O), 4.98 and 4.56 (2 d, J = 13, 13 Hz, OCH₂Ar), 3.40 and 3.26 (2 s, 2 CH₃O).

### Example 24

### 17-(Cyclopropylmethyl)-14-(2'-chlorobenzyloxy)-6,7-dehydro-4,5α-epoxy-3-hydroxy-6,7-2'-3'-indolomorphinan hydrochloride (compound 34).

M.p. > 250 °C (dec). 1 H NMR (DMSO-d6): δ 11.38 (s, NH), 9.38 (s, OH), 8.76 (broad s, ⁺NH), 7.34-6.85 (m, 8 arom. H), 6.72 (d, J = 8 Hz, 1 arom. H), 6.64 (d, J = 8 Hz, 1 arom. H), 5.93 (s, H-C(5)), 4.80 and 4.67 (2 d, J = 13, 13 Hz, OCH₂ Ar).

### Example 25

### Synthesis of 17-(Cyclopropylmethyl)-6,7-dehydro-3,14-dimethoxy-4,5α-epoxy-6,7-2'-3'-benzo[b]furanomorphinan (compound 35).

Sodium hydride (144 mg, 6 mmol; obtained from 240 mg of 60% sodium hydride dispersion in oil by washings with n-hexane) was added to a solution of naltriben methanesulfonate (P.S. Portoguese et al., J. Med. Chem., Vol. 34: 1715-1720, 1991) 500 mg, 0.97 mmol) in 10 ml of anhydrous N,N-dimethyl-formamide at 0 °C. The resulting mixture was stirred at 0 °C for 15 min and then at room temperature for another 30 min. After cooling to 0 °C, dimethyl sulfate (380 µl, 4 mmol) was added and stirring was continued at first at 0 °C for 30 min and then at room temperature for 3 h. Excess sodium hydride was destroyed by addition of MeOH and H₂O. The resulting mixture was extracted with ethyl acetate (3 x 40 ml), the combined organic layers were washed with H₂O (2 x 30 ml) and brine (2 x 30 ml), dried over Na₂SO₄ and evaporated to give a crystalline residue which was recrystallized from MeOH to afford 320 mg (74 %) of compound 35. M.p. 221-224 °C (dec.). 1 H NMR (CDCl₃): δ 7.47-7.14 (m, 4 arom. H), 6.64 (d, J =8.4 Hz, 1 arom. H), 6.59 (d, J = 8.4 Hz, 1 arom . H), 5.62 (s, H-C(5)), 3.78 (s, CH₃O-C(3)), 3.31 (s, CH₃O-C(14)).

### Example 26

### Synthesis of 17-Cyclopropylmethyl-6,7-dehydro-4,5α-epoxy-14-hydroxy-6,7-2',3'-benzo[b]furanomorphinan (compound 36).

A mixture of 3-deoxyonaltrexone (R. Krassnig and H. Schmidhammer, Heterocycles, Vol. 38: 877-881, 1994) (1,3 g, 3.99 mmol), O-phenylhydroxylamine hydrochloride (750 mg, 5.15 mmol), methanesulfonic acid (0.75 ml, 11.55 mmol), and ethanol (30 ml) was refluxed for 20 h. After cooling, the mixture was diluted with H₂O, alkalized with conc. NH₄OH and extracted with CH₂Cl₂ (4 x 40 ml). The combined organic layers were washed with H₂O (2 x 30 ml) and brine (30 ml), dried over Na₂SO₄ and evaporated to give a brownish oil which was crystallized form MeOH to yield 1.1 mg (69 %) of compound 36. M.p. > 260 °C. ¹H NMR (CDCl₃): δ 7.45 (d, J = 8 Hz, 1 arom. H), 7.37 (d, J = 8 Hz, 1 arom. H), 7.26-7.13 (m, 2 arom. H), 7.01 (dd, J = 7.8, 7.8 Hz, 1 arom. H), 6.67 (d, J = 7.8 Hz, 1 arom. H), 6.59 (d, J = 7.8 Hz, 1 arom. H), 5.59 (s, H-C(5)), 5.00 (broad s, OH).

### Example 27

### Synthesis of 17-(Cyclopropylmethyl)-6,7-dehydro-4,5α-epoxy-14-hydroxy-6,7-2'-3'-indolomorphinan hydrochloride (compound 37).

A mixture of 3-deoxyonaltrexone (R. Krassnig and H. Schmidhammer, Heterocycles, Vol. 38: 877-881, 1994) (1,5 g, 4.6 mmol), phenylhydrazine hydrochloride (1.0 mg, 6.9 mmol), 1M HCl in ether (5 ml), and methanol (20 ml) was stirred at room temperature for 3 days. After concentration to ca. half of the original volume in vacuo, the solution was refrigerated overnight. The colorless crystals formed were collcted to yield 1.54 g (77 %) of compound 37. M.p. > 240 °C (dec.). 1 H NMR (DMSO-d6): δ 11.37 (s, NH), 9.01 (broad s, ⁺NH), 7.36-6.94 (m, 5 arom. H), 6.78 (d, J = 7.8 Hz, 1 arom. H), 6.59 (d, J = 7.8 Hz, 1 arom. H), 6.55 (s, OH).

### Example 28

### 17(Cyclopropylmethyl)-6,7-dehydro-4,5α-epoxy-3-hydroxy-14-(3'-chlorobenzyloxy)-6,7,2',3'-benzo[b]furanomorphinan, hydrochloride (compound 39).

¹H NMR (DMSO-d6): δ 9.40 (s, OH), 8.59 (broad s, +NH), 7.53-6.90 (m, 8 arom. H), 6.65 (s, 2 arom. H), 6.03 (s, H-C(5)), 4.74 and 4.62 (2 d, J=13.6, 13.6 Hz, OCH₂(3'-CIPh)). Analysis calculated for C₃₃H₃₀ClNO₄. HCl. 1.5 H₂O: C 65.67, H 5.68, N 2.32; found: C 65.31, H 5.37, N 2.33.

### Example 29

### 17-(Cyclopropylmethyl)-6,7-dehydro-4,5α-epoxy-3-hydroxy-14-(2'-chlorobenzyloxy)-6,7,2',3'-benzo[b]furanomorphinan Hydrochloride (compound 41).

M.p. > 220°C. ¹H NMR (DMSO-d6): δ 9.40 (s, OH), 8.59 (broad s, +NH), 7.56-6.90 (m, 8 arom. H), 6.66 (m, 2 arom. H), 6.03 (s, H-C(5)), 4.74 (s, OCH₂(2-ClPh)). Analysis calculated for C₃₃H₃₀ClNO₄. Hcl. 1.5 H₂O: C 65.67, H 5.68, N, 2.32. Found: C 65.72, H 5.48, N 2.25.

### Example 30

### 14-Allyloxy-17-(cyclopropylmethyl)-6,7-dehydro-4,5α-epoxy-3-hydroxy-1'-allyl-6,7-2',3'-indolomorphinan hydrochloride (compound 42).

NMR of the free base (colorless oil)
¹H NMR (CDCl₃): δ 7.40 (d, J = 8.4 Hz, 1 arom. H), 7.24 (m, 1 arom. H), 7.15 (m, 1 arom. H), 7.03 (m, 1 arom. H), 6.57 (d, J = 8.4 Hz, 1 arom. H), 6.50 (d, J = 8.4 Hz, 1 arom. H), 6.08 (m, 1 olef. H), 5.76 (m, 1 olef. H), 5.72 (s, H-C(5)), 5.15-4.75 (m, 6 H, CH₂N,2 CH₂ = C), 4.24 and 3.92 (2 dd, J = 12.4, 4.8 Hz, CH₂O).
This free base was dissolved in ethyl ether and treated with HCl/ether solution HCl at 0°C. Isolation of the precipitate provided the title compound 42 as a solid.

### Pharmaceutical preparations

For the preparation of a pharmaceutical formulation, the active ingredient may be formulated to an injection, capsule, tablet, suppository, solution, ointment, cream, paste, plaster, patch. The pharmaceutical formulation may comprise the compound of formula (I) alone or may also comprise expedients such as stabilizers, buffering agents, diluents, isotonic agents, antiseptics. The pharmaceutical formulation may contain the above described active ingredient in the amount of 0.01-95 % by weight. The dose of the active ingredient may be appropriately selected depending on the objects of administration, administration route and conditions of the patients.

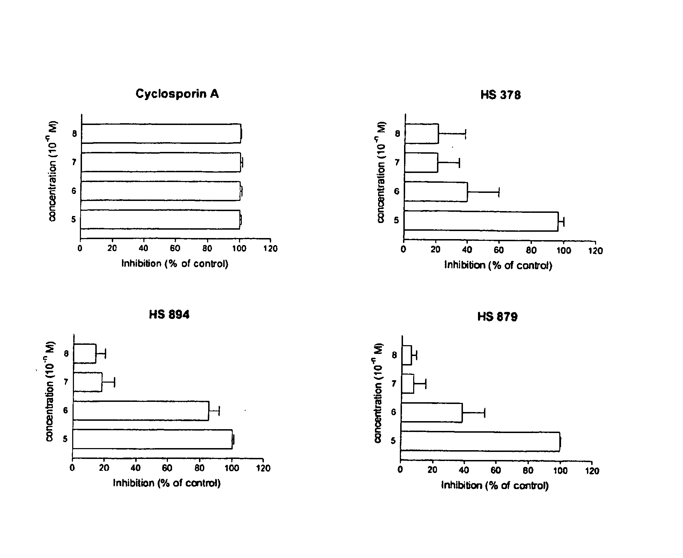

## Claims

1. Use of a compound according to the formula (I) 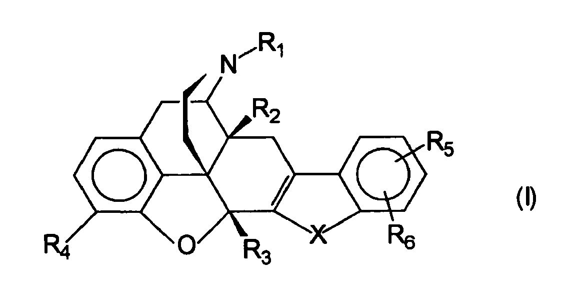 wherein
R₁ represents C₁-C₁₀ alkenyl; C₄-C₁₀ cycloalkylalkyl wherein the cycloalkyl is C₃-C₆ cycloalkyl and the alkyl is C₁-C₄ alkyl; C₄-C₁₀ cycloalkenylalkyl wherein the cycloalkenyl is C₃-C₆ cycloalkenyl and the alkyl is C₁-C₄ alkyl; C₇-C₁₆ arylalkyl wherein the aryl is C₆-C₁₀ aryl and the alkyl is C₁-C₆ alkyl; C₈-C₁₆ arylalkenyl wherein the aryl is C₆-C₁₀ aryl and the alkenyl is C₂-C₆ alkenyl;
R₂ represents hydrogen, hydroxy, C₁-C₆ alkoxy; C₁-C₆ alkenyloxy; C₇-C₁₆ arylalkyloxy wherein the aryl is C₆-C₁₀ aryl and the alkyloxy is C₁-C₆ alkyloxy; C₇-C₁₆ arylalkenyloxy wherein the aryl is C₆-C₁₀ aryl and the alkenyloxy is C₁-C₆ alkenyloxy; C₁-C₆ alkanoyloxy; C₇-C₁₆ arylalkanoyloxy wherein the aryl is C₆-C₁₀ aryl and the alkanoyloxy is C₁-C₆ alkanoyloxy;
R₃ represents hydrogen, C₁-C₆ alkyl; C₁-C₆ alkenyl; C₇-C₁₆ arylalkyl wherein the aryl is C₆-C₁₀ aryl and the alkyl is C₁-C₆ alkyl; C₇-C₁₆ arylalkenyl wherein the aryl is C₆-C₁₀ aryl and the alkenyl is C₁-C₆ alkenyl; hydroxy(C₁-C₆)alkyl; alkoxyalkyl wherein the alkoxy is C₁-C₆ alkoxy and the alkyl is C₁-C₆ alkyl; CO₂H; CO₂(C₁-C₆ alkyl);
R₄ is hydrogen, hydroxy; C₁-C₆ alkoxy; C₇-C₁₆ arylalkyloxy wherein the aryl is C₆-C₁₀ aryl and the alkyloxy is C₁-C₆ akyloxy; C₁-C₆ alkenyloxy; C₁-C₆ alkanoyloxy; C₇-C₁₆ arylalkanoyloxy wherein the aryl is C₆-C₁₀ aryl and the alkanoyloxy is C₁-C₆ alkanoyloxy; C₂-C₁₀ alkyloxyalkoxy wherein alkyloxy is C₁-C₄ alkyloxy and alkoxy is C₁-C₆ alkoxy;
R₅ and R₆ each independently represent hydrogen; OH; C₁-C₆ alkoxy; C₁-C₆ alkyl; hydroxyalkyl wherein the alkyl is C₁-C₆ alkyl; halo; nitro; cyano; thiocyanato; trifluoromethyl; CO₂H; CO₂(C₁-C₆ alkyl); CONH₂; CONH(C₁-C₆ alkyl); CON(C₁-C₆ alkyl)₂; amino; C₁-C₆ monoalkyl amino; C₁-C₆ dialkyl amino; C₅-C₆ cycloalkyl amino; SH; SO₃H; SO₃(C₁-C₆ alkyl); SO₂(C₁-C₆ alkyl); SO₂NH₂; SO₂NH(C₁-C₆ alkyl); SO₂NH (C₇-C₂₀ arylalkyl); SO(C₁-C₆ alkyl); or R₅ and R₆ together form a phenyl ring which may be unsubstituted or substituted by halo, nitro, cyano, thiocyanato; C₁-C₆ alkyl; trifluoromethyl; C₁-C₆ alkoxy, CO₂H, CO(C₁-C₆ alkyl), amino, C₁-C₆ monoalkylamino, C₁-C₆ dialkylamino, SH; SO₃H; H; SO₃(C₁-C₆ alkyl), SO₂(C₁-C₆ alkyl), SO(C₁-C₆ alkyl), and
X represents oxygen; sulfur; CH = CH or NR₉ wherein R₉ is H, C₁-C₆ alkyl, C₁-C₆ alkenyl, C₇-C₁₆ arylalkyl
wherein the aryl is C₆-C₁₀ aryl and the alkyl is C₁-C₆ alkyl, C₇-C₁₆ arylalkenyl wherein the aryl is C₆-C₁₀ aryl and the alkenyl is C₁-C₆ alkenyl; C₁-C₆ alkanoyl, and wherein aryl is unsubstituted or mono- or di- or trisubstituted independently with hydroxy, halo, nitro, cyano, thiocyanato, trifluoromethyl, C₁-C₃ alkyl, C₁-C₃ alkoxy, CO₂H, CO₂-(C₁-C₃)alkyl, CONH₂, CONH(C₁-C₃ alkyl), CON(C₁-C₃ alkyl), CO(C₁-C₃ alkyl), amino, (C₁-C₃ monoalkyl)amino, (C₁-C₃ dialkyl)amino, C₅-C₆ cycloalkylamino, (C₁-C₃ alkanoyl)amino, SH, SO₃H, SO₃ (C₁-C₃ alkyl), SO₂ (C₁-C₃ alkyl), SO(C₁-C₃ alkyl), C₁-C₃ alkylthio or C₁-C₃ alkanoylthio; and
with the proviso that when R₂ is hydroxy, R₃ cannot be hydrogen, except when R₄ is hydrogen, OCH₂OCH₃, OCH₂OC₂H₅ or OC(Ph)₃ and pharmacologically acceptable salts thereof, for the preparation of a medicament for the treatment and/or prevention of disorders susceptible to the inhibition of the target calcineurin, wherein the disorder is glaucoma and/or uveitis.

2. Use according to claim 1, wherein
R₁ is selected from allyl, cinnamyl, cyclopropylmethyl or cyclobutylmethyl;
R₂ is selected from methoxy, ethoxy, n-propyloxy, benzyloxy, benzyloxy substituted in the aromatic ring with F, Cl, NO₂, CN, CF₃, CH₃ or OCH₃; Allyloxy, cinnamyloxy or 3-phenylpropyloxy;
R₃ is selected from hydrogen, methyl, ethyl, benzyl or allyl;
R₄ is selected from hydroxy, methoxy, methoxymethoxy or acetyloxy;
R₅ and R₆ are each and independently selected from hydrogen; nitro; cyano; chloro, fluoro, bromo, trifluoromethyl; CO₂H; CO₂-CH₃; CONH₂; CONH₋CH₃; SH; SO₂NH₂; N(CH₃)₂; SO₂CH₃; X is selected from oxygen; NH or N CH₃, N-benzyl, N-allyl.

3. Use according to claim 1, wherein the compound of formula (I) is in the form of a pharmaceutically acceptable salt.

4. Use according to claim 1, wherein the salt is an inorganic salt.

5. Use according to claim 1, wherein the salt is an organic salt.

6. Use according to claim 1, wherein compound of formula (I) is
17-(Cyclopropylmethyl)-6,7-dehydro-4,5α-epoxy-1 4-ethoxy-3-hydroxy-5-methyl-6,7-2',3'-indolomorphinan x HCl;
17-Allyl-6,7-dehydro-4,5α-epoxy-14-ethoxy-3-hydroxy-5-methyl-6,7-2',3'-indolomorphinan x HCl;
6,7-Dehydro-4,5α-epoxy-14-ethoxy-3-hydroxy-5-methyl-17-(2-phenyl)ethyl-6,7-2',3'-indolomorphinan x HCl;
17-Allyl-6,7-dehydro-4,5α-epoxy-3-hydroxy-14-methoxy-5-methyl-6,7-2',3'-indolomorphinan x HCl;
6,7-Dehydro-4,5α-epoxy-3-hydroxy-14-methoxy-5-methyl-17-(2-phenyl)ethyl-6,7-2',3'-indolomorphinan x HCl;
17-(Cyclopropylmethyl)-6,7-dehydro-4,5α-epoxy-3-hydroxy-14-methoxy-5-methyl-6,7-2',3'-indolomorphinan x HCl;
17-Allyl-6,7-dehydro-4,5α-epoxy-3-hydroxy-5-methyl-14-n-propyloxy-6,7-2',3'-indolomorphinan x HCl
17-(Cyclopropylmethyl)-6,7-dehydro-4,5α-epoxy-3-hydroxy-5-methyl-14-n-propyloxy-6,7-2',3'-indolomorphinan x CH₃SO₃H;
17-(Cyclopropylmethyl)-6,7-dehydro-14-(2',6'-dichlorobenzyloxy)-4,5α-epoxy-3-(methoxymethoxy)-6,7-2',3'-benzo[b]furanomorphinan;
17-(Cyclopropylmethyl)-6,7-dehydro-14-(2',6'-dichlorobenzyloxy)-4,5α-epoxy-3-hydroxy-6,7-2',3'-benzo[b]furanomorphinan;
17-(Cyclopropylmethyl)-6,7-dehydro-4,5a-epoxy-3-(methoxymethoxy)-14-(3'-nitrobenzyloxy)-6,7-2',3'-benzo[b]furanomorphinan x HCl;
17-(Cyclopropylmethyl)-6,7-dehydro-4,5α-epoxy-3-hydroxy-14-(3'-nitrobenzyloxy)-6,7-2',3'-benzo[b]furanomorphinan x HCl;
17-(Cyclopropylmethyl)-6,7-dehydro-4,5α-epoxy-3-(methoxymethoxy)-14-(2'-naphthylmethoxy)-6,7-2',3'-benzo[b]furanomorphinan; 17-(Cyclopropylmethyl)-6,7-dehydro-4,5α-epoxy-3-hydroxy-14-(2'-naphthylmethoxy)-6,7-2',3'-benzo[b]furanomorphinan x HCl;
17-(Cyclopropylmethyl)-6,7-dehydro-4,5α-epoxy-14-(2'-fluorbenzyloxy)-3-(methoxymethoxy)-6,7-2',3'-benzo[b]furanomorphinan;
17-(Cyclopropylmethyl)-6,7-dehydro-4,5α-epoxy-14-(2'-fluorbenzyloxy)-3-hydroxy-6,7-2',3'-benzo[b]furanomorphinan x HCl;
14-Cinnamyloxy-17-(cyclopropylmethyl)-6,7-dehydro-4,5α-epoxy-3-(methoxymethoxy)-6,7-2'-3'-benzo[b]furanomorphinan;
14-Cinnamyloxy-17-(cyclopropylmethyl)-6,7-dehydro-4,5α-epoxy-3-hydroxy-6,7-2'-3'-benzo[b]furanomorphinan Salicylate;
17-(Cyclopropylmethyl)-6,7-dehydro-4,5α-epoxy-14-methoxy-3-(methoxymethoxy)-6,7-2'-3'-benzo[b]furanomorphinan;
17-(Cyclopropylmethyl)-14-(2'-chlorbenzyloxy)-6,7-dehydro-4,5α-epoxy-3-(methoxymethoxy)-6,7-2',3'-(N-methoxymethylindolo)morphinan;
17-(Cyclopropylmethyl)-14-(2'-chlorbenzyloxy)-6,7-dehydro-4,5α-epoxy-3-hydroxy-6,7-2',3'-indolomorphinan x HCl;
17(Cyclopropylmethyl)-6,7-dehydro-4,5α-epoxy-3-hydroxy-14-(3'-chlorbenzyloxy)-6,7,2',3'-benzo[b]furanomorphinan x HCl;
17-(Cyclopropylmethyl)-6,7-dehydro-4,5α-epoxy-3-hydroxy-14-(2'-chlorbenzyloxy)-6,7,2',3'-benzo[b]furanomorphinan x HCl; 14-Allyloxy-17-(cyclopropylmethyl)-6,7-dehydro-4,5α-epoxy-3-hydroxy-1'-allyl-6,7-2',3'-indolomorphinan x HCl;
17-(Cyctobutylmethyl)-6,7-didehydro-4,5α-epoxy-14β-ethoxy-5β-methylindolo[2',3':6,7]morphinan-3-ol Hydrochloride;
14ß-(Benzyloxy)-17-(cyclopropylmethyl)-6,7-didehydro-4,5α-epoxyindolo[2',3':6,7]morphinan-3-ol:
14ß-[(4-Chlorobenzyl)oxy]-17-(cyclopropylmethyl)-6,7-didehydro-4,5α-epoxyindolo[2',3':6,7]morphinan-3-ol Hydrochloride;
17-(Cyclopropylmethyl)-6,7-didehydro-4,5a-epoxy-14ß-[(2-phenylbenzyl)oxy]indolo[2',3':6,7]morphinan-3-ol Hydrochloride;
14ß-[(4-tert.-Butylbenzyl)oxy]-17-(cyclopropylmethyl)-6,7-didehydro-4,5α-epoxyindolo[2',3':6,7]morphinan-3-ol Hydrochloride;
17-(Cyclopropylmethyl)-6,7-didehydro-4,5α-epoxy-14ß-[(3-phenylpropyl)oxy]indolo[2',3':6,7]morphinan-3-ol.

7. Compound according to the formula (I) 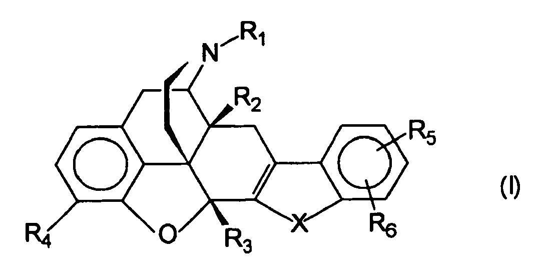 wherein
R₁ represents C₁-C₁₀ alkenyl; C₄-C₁₀ cycloalkylalkyl wherein the cycloalkyl is C₃-C₆ cycloalkyl and the alkyl is C₁-C₄ alkyl; C₄-C₁₀ cycloalkenylalkyl wherein the cycloalkenyl is C₃-C₆ cycloalkenyl and the alkyl is C₁-C₄ alkyl; C₇-C₁₆ arylalkyl wherein the aryl is C₆-C₁₀ aryl and the alkyl is C₁-C₆ alkyl; C₈-C₁₆ arylalkenyl wherein the aryl is C₆-C₁₀ aryl and the alkenyl is C₂-C₆ alkenyl;
R₂ represents hydrogen, hydroxy, C₁-C₆ alkoxy; C₁-C₆ alkenyloxy; C₇-C₁₆ arylalkyloxy wherein the aryl is C₆-C₁₀ aryl and the alkyloxy is C₁-C₆ alkyloxy; C₇-C₁₆ arylalkenyloxy wherein the aryl is C₅-C₁₀ aryl and the alkenyloxy is C₁-C₆ alkenyloxy; C₁-C₆ alkanoyloxy; C₇-C₁₆ arylalkanoyloxy wherein the aryl is C₆-C₁₀ aryl and the alkanoyloxy is C₁-C₆ alkanoyloxy;
R₃ represents hydrogen, C₁-C₆ alkyl; C₁-C₆ alkenyl; C₇-C₁₆ arylalkyl wherein the aryl is C₆-C₁₀ aryl and the alkyl is C₁-C₆ alkyl; C₇-C₁₆ arylalkenyl wherein the aryl is C₆-C₁₀ aryl and the alkenyl is C₁-C₆ alkenyl; hydroxy(C₁-C₆)alkyl; alkoxyalkyl wherein the alkoxy is C₁-C₆ alkoxy and the alkyl is C₁-C₆ alkyl; CO₂H; CO₂(C₁-C₆ alkyl);
R₄ is hydrogen, hydroxy; C₁-C₆ alkoxy; C₇-C₁₆ arylalkyloxy wherein the aryl is C₆-C₁₀ aryl and the alkyloxy is C₁-C₆ akyloxy; C₁-C₆ alkenyloxy; C₁-C₆ alkanoyloxy; C₇-C₁₆ arylalkanoyloxy wherein the aryl is C₆-C₁₀ aryl and the alkanoyloxy is C₁-C₆ alkanoyloxy; C₂-C₁₀ alkyloxyalkoxy wherein alkyloxy is C₁-C₄ alkyloxy and alkoxy is C₁-C₆ alkoxy;
R₅ and R₆ each independently represent hydrogen; OH; C₁-C₆ alkoxy; C₁-C₆ alkyl; hydroxyalkyl wherein the alkyl is C₁-C₆ alkyl; halo; nitro; cyano; thiocyanato; trifluoromethyl; CO₂H; CO₂(C₁-C₆ alkyl); CONH₂; CONH(C₁-C₆ alkyl); CON(C₁-C₆ alkyl)₂; amino; C₁-C₆ monoalkyl amino; C₁-C₆ dialkyl amino; C₅-C₆ cycloalkyl amino; SH; SO₃H; SO₃(C₁-C₆ alkyl); SO₂(C₁-C₆ alkyl); SO₂NH₂; SO₂NH(C₁-C₆ alkyl); SO₂NH (C₇-C₂₀ arylalkyl); SO(C₁-C₆ alkyl); or R₅ and R₆ together form a phenyl ring which may be unsubstituted or substituted by halo, nitro, cyano, thiocyanato; C₁-C₆ alkyl; trifluoromethyl; C₁-C₆ alkoxy, CO₂H, CO(C₁-C₆ alkyl), amino, C₁-C₆ monoalkylamino, C₁-C₆ dialkylamino, SH; SO₃H; H; SO₃(C₁-C₆ alkyl), SO₂(C₁-C₆ alkyl), SO(C₁-C₆ alkyl), and
X represents oxygen; sulfur; CH = CH or NR₉ wherein R₉ is H, C₁-C₆ alkyl, C₁-C₆ alkenyl, C₇-C₁₆ arylalkyl
wherein the aryl is C₆-C₁₀ aryl and the alkyl is C₁-C₆ alkyl, C₇-C₁₆ arylalkenyl wherein the aryl is C₆-C₁₀ aryl and the alkenyl is C₁-C₆ alkenyl; C₁-C₆ alkanoyl, and wherein aryl is unsubstituted or mono- or di- or trisubstituted independently with hydroxy, halo, nitro, cyano, thiocyanato, trifluoromethyl, C₁-C₃ alkyl, C₁-C₃ alkoxy, CO₂H. CO₂-(C₁-C₃)alkyl, CONH₂, CONH(C₁-C₃ alkyl), CON(C₁-C₃ alkyl), CO(C₁-C₃ alkyl), amino, (C₁-C₃ monoalkyl)amino, (C₁-C₃ dialkyl)amino, C₅-C₆ cycloalkylamino, (C₁-C₃ alkanoyl)amino, SH, SO₃H, SO₃ (C₁-C₃ alkyl), SO₂ (C₁-C₃ alkyl), SO(C₁-C₃ alkyl), C₁-C₃ alkylthio or C₁-C₃ alkanoylthio; and
with the proviso that when R₂ is hydroxy, R₃ cannot be hydrogen, except when R₄ is hydrogen, OCH₂OCH₃, OCH₂OC₂H₅ or OC(Ph)₃ and pharmacologically acceptable salts thereof, for use in the treatment and/or prevention of disorders susceptible to the inhibition of the target calcineurin,
wherein the disorder is glaucoma and/or uveitis.

8. Compound for use according to claim 7, wherein
R₁ is selected from allyl, cinnamyl, cyclopropylmethyl or cyclobutylmethyl;
R₂ is selected from methoxy, ethoxy, n-propyloxy, benzyloxy, benzyloxy substituted in the aromatic ring with F, Cl, NO₂, CN, CF₃, CH₃ or OCH₃; Allyloxy, cinnamyloxy or 3-phenylpropyloxy;
R₃ is selected from hydrogen, methyl, ethyl, benzyl or allyl;
R₄ is selected from hydroxy, methoxy, methoxymethoxy or acetyloxy;
R₅ and R₆ are each and independently selected from hydrogen; nitro; cyano; chloro, fluoro, bromo trifluoromethyl; CO₂H; CO₂ CH₃; CONH₂; CONH CH₃; SH; SO₂NH₂; N(CH₃)₂; SO₂CH₃; X is selected from oxygen; NH or N CH₃, N-benzyl, N-allyl.

9. Compound for use according to claim 7, wherein the compound of formula (I) is in the form of a pharmaceutically acceptable salt.

10. Compound for use according to claim 7, wherein the salt is an inorganic salt.

11. Compound for use according to claim 7, wherein the salt is an organic salt.

12. Compound for use according to claim 7, wherein compound of formula (I) is
17-(Cyclopropylmethyl)-6,7-dehydro-4,5α-epoxy-14-ethoxy-3-hydroxy-5-methyl-6,7-2',3'-indolomorphinan x HCl;
17-Allyl-6,7-dehydro-4,5α-epoxy-14-ethoxy-3-hydroxy-5-methyl-6,7-2', 3'-indolomorphinan x HCl;
6,7-Dehydro-4,5α-epoxy-14-ethoxy-3-hydroxy-5-methyl-17-(2-phenyl)ethyl-6,7-2',3'-indolomorphinan x HCl;
17-Allyl-6,7-dehydro-4,5α-epoxy-3-hydroxy-14-methoxy-5-methyl-6,7-2',3'-indolomorphinan x HCl;
6,7-Dehydro-4,5α-epoxy-3-hydroxy-14-methoxy-5-methyl-17-(2-phenyl)ethyl-6,7-2',3'-indolomorphinan x HCl;
17-(Cyclopropylmethyl)-6,7-dehydro-4,5α-epoxy-3-hydroxy-14-methoxy-5-methyl-6,7-2',3'-indolomorphinan x HCl;
17-Allyl-6,7-dehydro-4,5α-epoxy-3-hydroxy-5-methyl-14-n-propyloxy-6,7-2',3'-indolomorphinan x HCl
17-(Cyclopropylmethyl)-6,7-dehydro-4,5α-epoxy-3-hydroxy-5-methyl-14-n-propyloxy-6,7-2',3'-indolomorphinan x CH₃SO₃H;
17-(Cyclopropylmethyl)-6,7-dehydro-14-(2',6'-dichlorobenzyloxy)-4,5α-epoxy-3-(methoxymethoxy)-6,7-2',3'-benzo[b]furanomorphinan;
17-(Cyclopropylmethyl)-6,7-dehydro-14-(2',6'-dichlorobenzyloxy)-4,5α-epoxy-3-hydroxy-6,7-2',3'-benzo[b]furanomorphinan;
17-(Cyclopropylmethyl)-6,7-dehydro-4,5α-epoxy-3-(methoxymethoxy)-14-(3'-nitrobenzyloxy)-6,7-2',3'-benzo[b]furanomorphinan x HCl;
17-(Cyclopropylmethyl)-6,7-dehydro-4,5α-epoxy-3-hydroxy-14-(3'-nitrobenzyloxy)-6,7-2',3'-benzo[b]furanomorphinan x HCl
17-(Cyclopropylmethyl)-6,7-dehydro-4,5α-epoxy-3-(methoxymethoxy)-14-(2'-naphthylmethoxy)-6,7-2',3'-benzo[b]furanomorphinan;
17-(Cyclopropylmethyl)-6,7-dehydro-4,5α-expoxy-3-hydroxy-14-(2'-naphthylmethoxy)-6,7-2',3'-benzo[b]furanomorphinan x HCl;
17-(Cyclopropylmethyl)-6,7-dehydro-4,5α-epoxy-14-(2'-fluorbenzyloxy)-3-(methoxymethoxy)-6,7-2',3'-benzo[b]furanomorphinan;
17-(Cyclopropylmethyl)-6,7-dehydro-4,5α-epoxy-14-(2'-fluorbenzyloxy)-3-hydroxy-6,7-2',3'-benzo[b]furanomorphinan x HCl;
14-Cinnamyloxy-17-(cyclopropylmethyl)-6,7-dehydro-4,5α-epoxy-3-(methoxymethoxy)-6,7-2'-3'-benzo[b]furanomorphinan; 14-Cinnamyloxy-17-(cyclopropylmethyl)-6,7-dehydro-4,5α-epoxy-3-hydroxy-6,7-2'-3'-benzo[b]furanomorphinan Salicylate;
17-(Cyclopropylmethyl)-6,7-dehydro-4,5α-epoxy-14-methoxy-3-(methoxymethoxy)-6,7-2'-3'-benzo[b]furanomorphinan;
17-(Cyclopropylmethyl)-14-(2'-chlorbenzyloxy)-6,7-dehydro-4,5α-epoxy-3-(methoxymethoxy)-6,7-2',3'-(N-methoxymethylindolo)morphinan;
17-(Cyclopropylmethyl)-14-(2'-chlorbenzyloxy)-6,7-dehydro-4,5α-epoxy-3-hydroxy-6,7-2',3'-indolomorphinan x HCl;
17(Cyclopropylmethyl)-6,7-dehydro-4,5α-epoxy-3-hydroxy-14-(3'-chlorbenzyloxy)-6,7,2',3'-benzo[b]furanomorphinan x HCl;
17-(Cyclopropylmethyl)-6,7-dehydro-4,5α-epoxy-3-hydroxy-14-(2'-chlorbenzyloxy)-6,7,2',3'-benzo[b]furanomorphinan x HCl;
14-Allyloxy-17-(cyclopropylmethyl)-6,7-dehydro-4,5α-epoxy-3-hydroxy-1'-allyl-6,7-2',3'-indolomorphinan x HCl;
17-(Cyclobutylmethyl)-6,7-didehydro-4,5α-epoxy-14β-ethoxy-5β-methylindolo[2',3':6,7]morphinan-3-ol Hydrochloride;
14ß-(Benzyloxy)-17-(cyclopropylmethyl)-6,7-didehydro-4,5α-epoxyindolo[2',3':6,7]morphinan-3-ol:
14ß-[(4-Chlorobenzyl)oxy]-17-(cyclopropylmethyl)-6,7-didehydro-4,5α-epoxyindolo[2',3':6,7]morphinan-3-ol Hydrochloride;
17-(Cyclopropylmethyl)-6,7-didehydro-4,5α-epoxy-14ß-[(2-phenylbenzyl)oxy]indolo[2',3':6,7]morphinan-3-ol Hydrochloride; 14ß-[(4-tert.-Butylbenzyl)oxy]-17-(cyclopropylmethyl)-6,7-didehydro-4,5α-epoxyindolo[2',3':6,7]morphinan-3-ol Hydrochloride;
17-(Cyclopropylmethyl)-6,7-didehydro-4,5α-epoxy-14ß-[(3-phenylpropyl)oxy]indolo[2',3':6,7]morphinan-3-ol.

## Patentansprüche

1. Verwendung einer Verbindung gemäß der Formel (I) 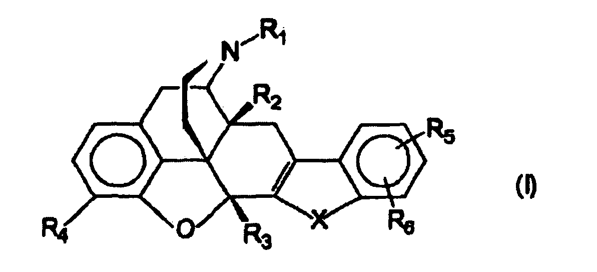 worin
R₁ C₁-C₁₀-Alkenyl; C₄-C₁₀-Cycloalkylalkyl, wobei das Cycloalkyl C₃-C₆-Cycloalkyl ist und das Alkyl C₁-C₄-Alkyl ist; C₄-C₁₀-Cycloalkenylalkyl, wobei das Cycloalkenyl C₃-C₆-Cycloalkenyl ist und das Alkyl C₁-C₄-Alkyl ist; C₇-C₁₆-Arylalkyl, wobei das Aryl C₆-C₁₀-Aryl ist und das Alkyl C₁-C₆-Alkyl ist; C₈-C₁₆-Arylalkenyl, wobei das Aryl C₆-C₁₀-Aryl ist und das Alkenyl C₂-C₆-Alkenyl ist, bedeutet;
R₂ Wasserstoff, Hydroxy, C₁-C₆-Alkoxy; C₁-C₆-Alkenyloxy; C₇-C₆-Arylalkyloxy, wobei das Aryl C₆-C₁₀-Aryl ist und das Alkyloxy C₁-C₆-Alkyloxy ist; C₇-C₁₆-Arylalkenyloxy, wobei das Aryl C₆-C₁₀-Aryl ist und das Alkenyloxy C₁-C₆-Alkenyloxy ist; C₁-C₆-Alkanoyloxy; C₇-C₁₆-Arylalkanoyloxy, wobei das Aryl C₆-C₁₀-Aryl ist und das Alkanoyloxy C₁-C₆-Alkanoyloxy ist, bedeutet;
R₃ Wasserstoff, C₁-C₆-Alkyl; C₇-C₆-Alkenyl; C₇-C₁₆-Arylalkyl, wobei das Aryl C₆-C₁₀-Aryl ist und das Alkyl C₁-C₆-Alkyl ist; C₇-C₁₆-Arylalkenyl, wobei das Aryl C₆-C₁₀-Aryl ist und das Alkenyl C₁-C₆-Alkenyl ist; Hydroxy(C₁-C₆)alkyl; Alkoxyalkyl, wobei das Alkoxy C₁-C₆-Alkoxy ist und das Alkyl C₁-C₆-Alkyl ist; CO₂H; CO₂(C₁-C₆-Alkyl) bedeutet;
R₄ Wasserstoff, Hydroxy; C₁-C₆-Alkoxy; C₇-C₁₆-Arylalkyloxy, wobei das Aryl C₆-C₁₀-Aryl ist und das Alkyloxy C₁-C₆-Alkyloxy ist; C₁-C₆-Alkenyloxy; C₁-C₆-Alkanoyloxy; C₇-C₁₆-Arylalkanoyloxy, wobei das Aryl C₆-C₁₀-Aryl ist und das Alkanoyloxy C₁-C₆-Alkanoyloxy ist; C₂-C₁₀-Alkyloxyalkoxy, wobei Alkyloxy C₁-C₄-Alkyloxy ist und Alkoxy C₁-C₆-Alkoxy ist, ist;
R₅ und R₆ jeweils unabhängig voneinander Wasserstoff; OH; C₁-C₆-Alkoxy; C₁-C₆-Alkyl; Hydroxyalkyl, wobei das Alkyl C₁-C₆-Alkyl ist; Halogen; Nitro; Cyano; Thiocyanato; Trifluormethyl; CO₂H; CO₂(C₁-C₆-Alkyl); CONH₂; CONH(C₁-C₆-Alkyl); CON(C₁-C₆-Alkyl)₂; Amino; C₁-C₆-Monoalkylamino; C₁-C₆-Dialkylamino; C₅-C₆-Cycloalkylamino; SH; SO₃H; SO₃(C₁-C₆-Alkyl); SO₂(C₁-C₆-Alkyl); SO₂NH₂; SO₂NH(C₁-C₆-Alkyl); SO₂NH(C₇-C₂₀-Arylalkyl); SO(C₁-C₆-Alkyl) bedeuten; oder R₅ und R₆ zusammen einen Phenylring bilden, welcher unsubstituiert oder durch Halogen, Nitro, Cyano, Thiocyanato; C₁-C₆-Alkyl; Trifluormethyl; C₁-C₆-Alkoxy, CO₂H, CO(C₁-C₆-Alkyl), Amino, C₁-C₆-Monoalkylamino, C₁-C₆-Dialkylamino, SH; SO₃H; H; SO₃(C₁-C₆-Alkyl), SO₂(C₁-C₆-Alkyl), SO(C₁-C₆-Alkyl) substituiert sein kann, und
X Sauerstoff; Schwefel; CH=CH oder NR₉ bedeutet, wobei R₉ H, C₁-C₆-Alkyl, C₁-C₆-Alkenyl, C₇-C₁₆-Arylalkyl, wobei das Aryl C₆-C₁₀-Aryl ist und das Alkyl C₁-C₆-Alkyl ist, C₇-C₁₆-Arylalkerlyl, wobei das Aryl C₆-C₁₀-Aryl ist und das Alkenyl C₁-C₆-Alkenyl ist; C₁-C₆-Alkanoyl ist, und wobei Aryl unsubstituiert oder unabhängig voneinander mit Hydroxy, Halogen, Nitro, Cyano, Thiocyanato, Trifluormethyl, C₁-C₃-Alkyl, C₁-C₃-Alkoxy, CO₂H, CO₂(C₁-C₃)Alkyl, CONH₂, CONH(C₁-C₃-Alkyl), CON(C₁-C₃-Alkyl), CO(C₁-C₃-Alkyl), Amino, (C₁-C₃-Monoalkyl)amino, (C₁-C₃-Dialkyl)amino, C₅-C₆-Cycloalkylamino, (C₁-C₃-Alkanoyl)amino, SH, SO₃H, SO₃(C₁-C₃-Alkyl), SO₂(C₁-C₃-Alkyl), SO(C₁-C₃-Alkyl), C₁-C₃-Alkylthio oder C₁-C₃-Alkanoylthlo mono- oder di- oder trisubstituiert ist; und
mit der Maßgabe, dass, wenn R₂ Hydroxy ist, R₃ nicht Wasserstoff sein kann, außer wenn R₄ Wasserstoff, OCH₂OCH₃, OCH₂OC₂H₅ oder OC(Ph)₃ ist, und von pharmakologisch verträglichen Salzen davon, für die Herstellung eines Medikamentes für die Behandlung und/oder Verhütung von Störungen, die für die Hemmung des Targets Calcineurin empfänglich sind, wobei es sich bei der Störung um ein Glaukom und/oder Uveitis handelt.

2. Verwendung nach Anspruch 1, wobei
R₁ ausgewählt ist aus Allyl, Cinnamyl, Cyclopropylmethyl oder Cyclobutylmethyl;
R₂ ausgewählt ist aus Methoxy, Ethoxy, n-Propyloxy, Benzyloxy, Benzyloxy, das in dem aromatischen Ring mit F, Cl, NO₂, CN, CF₃, CH₃ oder OCH₃ substituiert ist; Allyloxy, Cinnamyloxy oder 3-Phenylpropyloxy;
R₃ ausgewählt ist aus Wasserstoff, Methyl, Ethyl, Benzyl oder Allyl;
R₄ ausgewählt ist aus Hydroxy, Methoxy, Methoxymethoxy oder Acetyloxy;
R₅ und R₆ jeweils und unabhängig voneinander ausgewählt sind aus Wasserstoff; Nitro; Cyano; Chlor, Fluor, Brom, Trifluormethyl; CO₂H; CO₂-CH₃; CONH₂; CONH-CH₃; SH; SO₂NH₂; N(CH₃)₂; SO₂CH₃; X ausgewählt ist aus Sauerstoff; NH oder NCH₃, N-Benzyl, N-Allyl.

3. Verwendung nach Anspruch 1, wobei die Verbindung der Formel (I) in Form eines pharmazeutisch verträglichen Salzes vorliegt.

4. Verwendung nach Anspruch 1, wobei das Salz ein anorganisches Salz ist.

5. Verwendung nach Anspruch 1, wobei das Salz ein organisches Salz ist.

6. Verwendung nach Anspruch 1, wobei die Verbindung der Formel (I)
17-(Cyclopropylmethyl)-6,7-dehydro-4,5α-epoxy-14-ethoxy-3-hydroxy-5-methyl-6,7-2',3'-indolomorphinan x HCl;
17-Allyl-6,7-dehydro-4,5α-epoxy-14-ethoxy-3-hydroxy-5-methyl-6,7-2',3'-indolomorphinan x HCl;
6,7-Dehydro-4,5α-epoxy-14-ethoxy-3-hydroxy-5-methyl-17-(2-phenyl)ethyl-6,7-2',3'-indolomorphinan x HCl;
17-Allyl-6,7-dehydro-4,5α-epoxy-3-hydroxy-14-methoxy-5-methyl-6,7-2',3'-indolomorphinan x HCl;
6,7-Dehydro-4,5α-epoxy-3-hydroxy-14-methoxy-5-methyl-17-(2-phenyl)ethyl-6,7-2',3'-indolomorphinan x HCl;
17-(Cyclopropylmethyl)-6,7-dehydro-4,5α-epoxy-3-hydroxy-14-methoxy-5-methyl-6,7-2',3'-indolomorphinan x HCl;
17-Allyl-6,7-dehydro-4,5α-epoxy-3-hydroxy-5-methyl-14-n-propyloxy-6,7-2',3'-indolomorphinan x HCl;
17-(Cyclopropylmethyl)-6,7-dehydro-4,5α-epoxy-3-hydroxy-5-methyl-14-n-propyloxy-6,7-2',3'-indolomorphinan x CH₃SO₃H;
17-(Cyclopropylmethyl)-6,7-dehydro-14-(2',6'-dichlorbenzyloxy)-4,5α-epoxy-3-(methoxymethoxy)-6,7-2',3'-benzo[b]furanomorphinan;
17-(Cyclopropylmethyl)-6,7-dehydro-14-(2',6'-dichlorbenzyloxy)-4,5α-epoxy-3-hydroxy-6,7-2',3'-benzo[b]furanomorphinan;
17-(Cyclopropylmethyl)-6,7-dehydro-4,5α-epoxy-3-(methoxymethoxy)-14-(3'-nitrobenzyloxy)-6,7-2',3'-benzo[b]furanomorphinan x HCl;
17-(Cyclopropylmethyl)-6,7-dehydro-4,5α-epoxy-3-hydroxy-14-(3'-nitrobenzyloxy)-6,7-2',3'-benzo[b]furanomorphinan x HCl;
17-(Cyclopropylmethyl)-6,7-dehydro-4,5α-epoxy-3-(methoxymethoxy)-14-(2'-naphthylmethoxy)-6,7-2',3'-benzo[b]furanomorphinan;
17-(Cyclopropylmethyl)-6,7-dehydro-4,5α-epoxy-3-hydroxy-14-(2'-naphthylmethoxy)-6,7-2',3'-benzo[b]furanomorphinan x HCl;
17-(Cyclopropylmethyl)-6,7-dehydro-4,5α-epoxy-14-(2'-fluorbenzyloxy)-3-(methoxymethoxy)-6,7-2',3'-benzo[b]furanomorphinan;
17-(Cyclopropylmethyl)-6,7-dehydro-4,5α-epoxy-14-(2'-fluorbenzyloxy)-3-hydroxy-6,7-2',3'-benzo[b]furanomorphinan x HCl;
14-Cinnamyloxy-17-(cyclopropylmethyl)-6,7-dehydro-4,5α-epoxy-3-(methoxymethoxy)-6,7-2'-3'-benzo[b]furanomorphinan;
14-Cinnamyloxy-17-(cyclopropylmethyl)-6,7-dehydro-4,5α-epoxy-3-hydroxy-6,7-2'-3'-benzo[b]furanomorphinan-Salicylat;
17-(Cyclopropylmethyl)-6,7-dehydro-4,5α-epoxy-14-methoxy-3-(methoxymethoxy)-6,7-2'-3'-benzo[b]furanomorphinan;
17-(Cyclopropylmethyl)-14-(2'-chlorbenzyloxy)-6,7-dehydro-4,5α-epoxy-3-(methoxymethoxy)-6,7-2',3'-(N-methoxymethylindolo)morphinan;
17-(Cyclopropylmethyl)-14-(2'-chlorbenzyloxy)-6,7-dehydro-4,5α-epoxy-3-hydroxy-6,7-2',3'-indolomorphinan x HCl;
17-(Cyclopropylmethyl)-6,7-dehydro-4,5α-epoxy-3-hydroxy-14-(3'-chlorbenzyloxy)-6,7,2',3'-benzo[b]furanomorphinan x HCl;
17-(Cyclopropylmethyl)-6,7-dehydro-4,5α-epoxy-3-hydroxy-14-(2'-chlorbenzyloxy)-6,7,2',3'-benzo[b]furanomorphinan x HCl;
14-Allyloxy-17-(cyclopropylmethyl)-6,7-dehydro-4,5α-epoxy-3-hydroxy-1'-allyl-6,7-2',3'-indolomorphinan x HCl;
17-(Cyclobutylmethyl)-6,7-didehydro-4,5α-epoxy-14β-ethoxy-5β-methylindolo[2',3':6,7]morphinan-3-ol-Hydrochlorid;
14β-(Benzyloxy)-17-(cyclopropylmethyl)-6,7-didehydro-4,5α-epoxyindolo[2',3':6,7]morphinan-3-ol:
14β-[(4-Chlorbenzyl)oxy]-17-(cyclopropylmethyl)-6,7-didehydro-4,5α-epoxyindolo[2',3':6,7]morphinan-3-ol-Hydrochlorid;
17-(Cyclopropylmethyl)-6,7-didehydro-4,5α-epoxy-14β-[(2-phenylbenzyl)oxy]indolo[2',3':6,7]morphinan-3-ol-Hydrochlorid;
14β-[(4-tert.-Butylbenzyl)oxy]-17-(cyclopropylmethyl)-6,7-didehydro-4,5α-epoxyindolo[2',3':6,7]morphinan-3-ol-Hydrochlorid;
17-(Cyclopropylmethyl)-6,7-didehydro-4,5α-epoxy-14β-[(3-phenylpropyl)oxy]indolo[2',3':6,7]morphinan-3-ol ist.

7. Verbindung nach Formel (I) 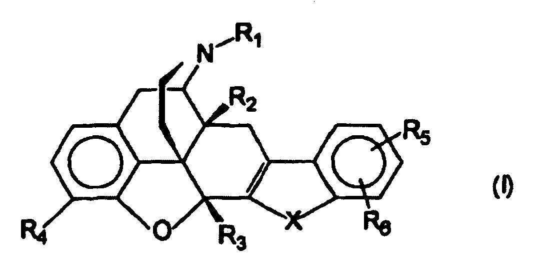 worin
R₁ C₁-C₁₀-Alkenyl; C₄-C₁₀-Cycloalkylalkyl, wobei das Cycloalkyl C₃-C₆-Cycloalkyl ist und das Alkyl C₁-C₄-Alkyl ist; C₄-C₁₀-Cycloalkenylalkyl, wobei das Cycloalkenyl C₃-C₆-Cycloalkenyl ist und das Alkyl C₁-C₄-Alkyl ist; C₇C₁₆.Arylalkyl, wobei das Aryl C₆-C₁₀-Aryl ist und das Alkyl C₁-C₆-Alkyl ist; C₈-C₁₆-Arylalkenyl, wobei das Aryl C₆-C₁₀-Aryl ist und das Alkenyl C₂-C₆-Alkenyl ist, bedeutet;
R₂ Wasserstoff, Hydroxy, C₁-C₆-Alkoxy; C₁-C₆-Alkenyloxy; C₇-C₁₆-Arylalkyloxy, wobei das Aryl C₆-C₁₀-Aryl ist und das AlkyloxyC₁-C₆-Alkyloxy ist; C₇-C₁₆-Arylalkenyloxy, wobei das Aryl C₆-C₁₀-Aryl ist und das Alkenyloxy C₁-C₆-Alkenyloxy ist; C₁-C₆-Alkanoyloxy; C₇-C₁₆-Arylalkanoyloxy, wobei das Aryl C₆-C₁₀-Aryl ist und das Alkanoyloxy C₁-C₆-Alkanoyloxy ist, bedeutet;
R₃ Wasserstoff, C₁-C₆-Alkyl; C₁-C₆-Alkenyl; C₇-C₁₆-Arylalkyl, wobei das Aryl C₆-C₁₀-Aryl ist und das Alkyl C₁-C₆-Alkyl ist; C₇-C₁₆-Arylalkenyl, wobei das Aryl C₆-C₁₀-Aryl ist und das Alkenyl C₁-C₆-Alkenyl ist; Hydroxy(C₁-C₆)alkyl; Alkoxyalkyl, wobei das Alkoxy C₁-C₆-Alkoxy ist und das Alkyl C₁-C₆-Alkyl ist; CO₂H; CO₂(C₁-C₆-Alkyl) bedeutet;
R₄ Wasserstoff, Hydroxy; C₁-C₆-Alkoxy; C₇-C₁₆-Arylalkyloxy, wobei das Aryl C₆-C₁₀-Aryl ist und das Alkyloxy C₁-C₆-Alkyloxy ist; C₁-C₆-Alkenyloxy; C₁-C₆-Alkanoyloxy; C₇-C₁₆-Arylalkanoyloxy, wobei das Aryl C₆-C₁₀-Aryl ist und das Alkanoyloxy C₁-C₆-Alkanoyloxy ist; C₂-C₁₀-Alkyloxyalkoxy, wobei Alkyloxy C₁-C₄-Alkyloxy ist und Alkoxy C₁-C₆-Alkoxy ist, ist;
R₅ und R₆ jeweils unabhängig voneinander Wasserstoff; OH; C₁-C₆-Alkoxy; C₁-C₆-Alkyl; Hydroxyalkyl, wobei das Alkyl C₁-C₆-Alkyl ist; Halogen; Nitro; Cyano; Thiocyanato; Trifluormethyl; CO₂H; CO₂(C₁-C₆-Alkyl); CONH₂; CONH(C₁-C₆-Alkyl); CON(C₁-C₆-Alkyl)₂; Amino; C₁-C₆-Monoalkylamino; C₁-C₆-Dialkylamino; C₅-C₆-Cycloalkylamino; SH; SO₃H; SO₃(C₁-C₆-Alkyl); SO₂(C₁-C₆-Alkyl); SO₂NH₂; SO₂NH(C₁-C₆-Alkyl); SO₂NH(C₇-C₂₀-Arylalkyl); SO(C₁-C₆-Alkyl) bedeuten; oder R₅ und R₆ zusammen einen Phenylring bilden, welcher unsubstituiert oder durch Halogen, Nitro, Cyano, Thiocyanato; C₁-C₆-Alkyl; Trifluormethyl; C₁-C₆-Alkoxy, CO₂H, CO(C₁-C₆-Alkyl), Amino, C₁-C₆-Monoalkylamino, C₁-C₆-Dialkylamino, SH; SO₃H; H; SO₃(C₁-C₆-Alkyl), SO₂(C₁-C₆-Alkyl), SO(C₁-C₆-Alkyl) substituiert sein kann, und
X Sauerstoff; Schwefel; CH=CH oder NR₉ bedeutet, wobei R₉ H, C₁-C₆-Alkyl, C₁-C₆-Alkenyl, C₇-C₁₆-Arylalkyl, wobei das Aryl C₆-C₁₀-Aryl ist und das Alkyl C₁-C₆-Alkyl ist, C₇-C₁₆-Arylalkenyl, wobei das Aryl C₆-C₁₀-Aryl ist und das Alkenyl C₁-C₆-Alkenyl ist; C₁-C₆-Alkanoyl ist, und wobei Aryl unsubstituiert oder unabhängig voneinander mit Hydroxy, Halogen, Nitro, Cyano, Thiocyanato, Trifluormethyl, C₁-C₃-Alkyl, C₁-C₃-Alkoxy, CO₂H, CO₂(C₁-C₃)Alkyl, CONH₂, CONH(C₁-C₃-Alkyl), CON(C₁-C₃-Alkyl), CO(C₁-C₃-Alkyl), Amino, (C₁-C₃-Monoalkyl)amino, (C₁-C₃-Dialkyl)amino, C₅-C₆-Cycloalkylamino, (C₁-C₃-Alkanoyl)amino, SH, SO₃H, SO₃(C₁-C₃-Alkyl), SO₂(C₁-C₃-Alkyl), SO(C₁-C₃-Alkyl), C₁-C₃-Alkylthio oder C₁-C₃-Alkanoylthio mono- oder di- oder trisubstituiert ist; und mit der Maßgabe, dass, wenn R₂ Hydroxy ist, R₃ nicht Wasserstoff sein kann, außer wenn R₄ Wasserstoff, OCH₂OCH₃, OCH₂OC₂H₅ oder OC(Ph)₃ ist, und pharmakologisch verträgliche Salze davon, zur Verwendung bei der Behandlung und/oder Verhütung von Störungen, die für die Hemmung des Targets Calcineurin empfänglich sind, wobei es sich bei der Störung um ein Glaukom und/oder Uveitis handelt.

8. Verbindung zur Verwendung nach Anspruch 7, wobei
R₁ ausgewählt ist aus Allyl, Cinnamyl, Cyclopropylmethyl oder Cyclobutylmethyl;
R₂ ausgewählt ist aus Methoxy, Ethoxy, n-Propyloxy, Benzyloxy, Benzyloxy, das in dem aromatischen Ring mit F, Cl, NO₂, CN, CF₃, CH₃ oder OCH₃ substituiert ist; Allyloxy, Cinnamyloxy oder 3-Phenylpropyloxy;
R₃ ausgewählt ist aus Wasserstoff, Methyl, Ethyl, Benzyl oder Allyl;
R₄ ausgewählt ist aus Hydroxy, Methoxy, Methoxymethoxy oder Acetyloxy;
R₅ und R₆ jeweils und unabhängig voneinander ausgewählt sind aus Wasserstoff; Nitro; Cyano; Chlor, Fluor, Brom, Trifluormethyl; CO₂H; CO₂-CH₃; CONH₂; CONH-CH₃; SH; SO₂NH₂; N(CH₃)₂; SO₂CH₃; X ausgewählt ist aus Sauerstoff; NH oder NCH₃, N-Benzyl, N-Allyl.

9. Verbindung zur Verwendung nach Anspruch 7, wobei die Verbindung der Formel (I) in Form eines pharmazeutisch verträglichen Salzes vorliegt.

10. Verbindung zur Verwendung nach Anspruch 7, wobei das Salz ein anorganisches Salz ist.

11. Verbindung zur Verwendung nach Anspruch 7, wobei das Salz ein organisches Salz ist.

12. Verbindung zur Verwendung nach Anspruch 7, wobei die Verbindung der Formel (I)
17-(Cyclopropylmethyl)-6,7-dehydro-4,5α-epoxy-14-ethoxy-3-hydroxy-5-methyl-6,7-2',3'-indolomorphinan x HCl;
17-Allyl-6,7-dehydro-4,5α-epoxy-14-ethoxy-3-hydroxy-5-methyl-6,7-2',3'-indolomorphinan x HCl;
6,7-Dehydro-4,5α-epoxy-14-ethoxy-3-hydroxy-5-methyl-17-(2-phenyl)ethyl-6,7-2',3'-indolomorphinan x HCl;
17-Allyl-6,7-dehydro-4,5α-epoxy-3-hydroxy-14-methoxy-5-methyl-6,7-2',3'-indolomorphinan x HCl;
6,7-Dehydro-4,5α-epoxy-3-hydroxy-14-methoxy-5-methyl-17-(2-phenyl)ethyl-6,7-2',3'-indolomorphinan x HCl;
17-(Cyclopropylmethyl)-6,7-dehydro-4,5α-epoxy-3-hydroxy-14-methoxy-5-methyl-6,7-2',3'-indolomorphinan x HCl;
17-Allyl-6,7-dehydro-4,5α-epoxy-3-hydroxy-5-methyl-1 4-n-propyloxy-6,7-2',3'-indolomorphinan x HCl;
17-(Cyclopropylmethyl)-6,7-dehydro-4,5α-epoxy-3-hydroxy-5-methyl-14-n-propyloxy-6,7-2',3'-indolomorphinan x CH₃SO₃H;
17-(Cyclopropylmethyl)-6,7-dehydro-14-(2',6'-dichlorbenzyloxy)-4,5α-epoxy-3-(methoxymethoxy)-6,7-2',3'-benzo[b]furanomorphinan;
17-(Cyclopropylmethyl)-6,7-dehydro-14-(2',6'-dichlorbenzyloxy)-4,5α-epoxy-3-hydroxy-6,7-2',3'-benzo[b]furanomorphinan;
17-(Cyclopropylmethyl)-6,7-dehydro-4,5α-epoxy-3-(methoxymethoxy)-14-(3'-nitrobenzyloxy)-6,7-2',3'-benzo[b]furanomorphinan x HCl;
17-(Cyclopropylmethyl)-6,7-dehydro-4,5α-epoxy-3-hydroxy-1 4-(3-nitrobenzyloxy)-6,7-2',3'-benzo[b]furanomorphinan x HCl;
17-(Cyclopropylmethyl)-6,7-dehydro-4,5α-epoxy-3-(methoxymethoxy)-14-(2'-naphthylmethoxy)-6,7-2',3'-benzo[b]furanomorphinan;
17-(Cyclopropylmethyl)-6,7-dehydro-4,5α-epoxy-3-hydroxy-14-(2'-naphthylmethoxy)-6,7-2',3'-benzo[b]furanomorphinan x HCl;
17-(Cyclopropylmethyl)-6,7-dehydro-4,5α-epoxy-14-(2'-fluorbenzyloxy)-3-(methoxymethoxy)-6,7-2',3'-benzo[b]furanomorphinan;
17-(Cyclopropylmethyl)-6,7-dehydro-4,5α-epoxy-14-(2'-fluorbenzyloxy)-3-hydroxy-6,7-2',3'-benzo[b]furanomorphinan x HCl;
14-Cinnamyloxy-17-(cyclopropylmethyl)-6,7-dehydro-4,5α-epoxy-3-(methoxymethoxy)-6,7-2'-3'-benzo[b]furanomorphinan;
14-Cinnamyloxy-17-(cyclopropylmethyl)-6,7-dehydro-4,5α-epoxy-3-hydroxy-6,7-2'-3'-benzo[b]furanomorphinan-Salicylat;
17-(Cyclopropylmethyl)-6,7-dehydro-4,5α-epoxy-14-methoxy-3-(methoxymethoxy)-6,7-2'-3'-benzo[b]furanomorphinan;
17-(Cyclopropylmethyl)-14-(2'-chlorbenzyloxy)-6,7-dehydro-4,5α-epoxy-3-(methoxymethoxy)-6,7-2',3'-(N-methoxymethylindolo)morphinan;
17-(Cyclopropylmethyl)-14-(2'-chlorbenzyloxy)-6,7-dehydro-4,5α-epoxy-3-hydroxy-6,7-2',3'-indolomorphinan x HCl;
17-(Cyclopropylmethyl)-6,7-dehydro-4,5α-epoxy-3-hydroxy-14-(3'-chlorbenzyloxy)-6,7,2',3'-benzo[b]furanomorphinan x HCl;
17-(Cyclopropylmethyl)-6,7-dehydro-4,5α-epoxy-3-hydroxy-14-(2'-chlorbenzyloxy)-6,7,2',3'-benzo[b]furanomorphinan x HCl;
14-Allyloxy-17-(cyclopropylmethyl)-6,7-dehydro-4,5α-epoxy-3-hydroxy-1'-allyl-6,7-2',3'-indolomorphinan x HCl;
17-(Cyclobutylmethyl)-6,7-didehydro-4,5α-epoxy-14β-ethoxy-5β-methylindolo[2',3':6,7]morphinan-3-ol-Hydrochlorid;
14β-(Benzyloxy)-17-(cyclopropylmethyl)-6,7-didehydro-4,5α-epoxyindolo[2',3':6,7]morphinan-3-ol:
14β-[(4-Chlorbenzyl)oxy]-17-(cyclopropylmethyl)-6,7-didehydro-4,5α-epoxyindolo[2',3':6,7]morphinan-3-ol-Hydrochlorid;
17-(Cyclopropylmethyl)-6,7-didehydro-4,5α-epoxy-14β-[(2-phenylbenzyl)oxy]indolo[2',3':6,7]morphinan-3-ol-Hydrochlorid;
14β-[(4-tert.-Butylbenzyl)oxy]-17-(cyclopropylmethyl)-6,7-didehydro-4,5α-epoxyindolo[2',3':6,7]morphinan-3-ol-Hydrochlorid;
17-(Cyclopropylmethyl)-6,7-didehydro-4,5α-epoxy-14β-[(3-phenylpropyl)oxy]indolo[2',3':6,7]morphinan-3-ol ist.

## Revendications

1. Utilisation d'un composé de formule (I) 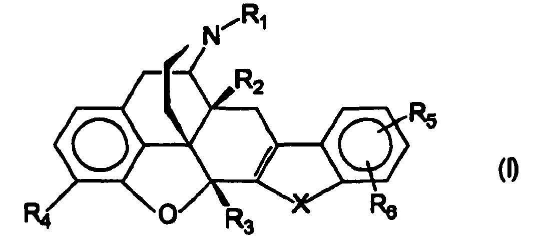 dans laquelle
R₁ représente un alcényle en C₁-C₁₀; un cycloalkylalkyle en C₄-C₁₀ où le cycloalkyle est un cycloalkyle en C₃-C₆ et l'alkyle est un alkyle en C₁-C₄; un cycloalcénylalkyle en C₄-C₁₀ où le cycloalcényle est un cycloalcényle en C₃-C₆ et l'alkyle est un alkyle en C₁-C₄; un arylalkyle en C₇-C₁₆ où l'aryle est un aryle en C₆-C₁₀ et l'alkyle est un alkyle en C₁-C₆; un arylalcényle en C₈-C₁₆ où l'aryle est un aryle en C₆-C₁₀ et l'alcényle est un alcényle en C₂-C₆;
R₂ représente l'hydrogène, un hydroxy, un alcoxy en C₁-C₆; un alcényloxy en C₁-C₆; un arylalkyloxy en C₇-C₁₆ où l'aryle est un aryle en C₆-C₁₀ et l'alkyloxy est un alkyloxy en C₁-C₆; un arylalcényloxy en C₇-C₁₆ où l'aryle et un aryle en C₆-C₁₀ et l'alcényloxy est un alcényloxy en C₁-C₆; un alcanoyloxy en C₁-C₆; un arylalcanoyloxy en C₇-C₁₆ où l'aryle est un aryle en C₆-C₁₀ et l'alcanoyloxy est un alcanoyloxy en C₁-C₆;
R₃ représente l'hydrogène, un alkyle en C₁-C₆; un alcényle en C₁-C₆; un arylalkyle en C₇-C₁₆ où l'aryle est un aryle en C₆-C₁₀ et l'alkyle est un alkyle en C₁-C₆; un arylalcényle en C₇-C₁₆ où l'aryle est un aryle en C₆-C₁₀ et l'alcényle est un alcényle en C₁-C₆; un hydroxyalkyle(en C₁-C₆); un alcoxyalkyle où l'alcoxy est un alcoxy en C₁-C₆ et l'alkyle est un alkyle en C₁-C₆; CO₂H; CO₂ (alkyle en C₁-C₆);
R₄ est l'hydrogène, un hydroxy; un alcoxy en C₁-C₆; un arylalkyloxy en C₇-C₁₆ où l'aryle est un aryle en C₆-C₁₀ et l'alkyloxy est un alkyloxy en C₁-C₆; un alcényloxy en C₁-C₆; un alcanoyloxy en C₁-C₆; un arylalcanoyloxy en C₇-C₁₆ où l'aryle est un aryle en C₆-C₁₀ et l'alcanoyloxy est un alcanoyloxy en C₁-C₆; un alkyloxyalcoxy en C₂-C₁₀ où l'alkyloxy est un alkyloxy en C₁-C₄ et l'alcoxy est un alcoxy en C₁-C₆;
R₅ et R₆ représentent chacun indépendamment l'hydrogène; OH; un alcoxy en C₁-C₆; un alkyle en C₁-C₆; un hydroxyalkyle où l'alkyle est un alkyle en C₁-C₆; un halogène; un nitro; un cyano; un thiocyanato; un trifluorométhyle; CO₂H; CO₂ (alkyle en C₁-C₆) ; CONH₂; CONH(alkyle en C₁-C₆); CON(alkyle en C₁-C₆)₂; un amino; un monoalkylamino en C₁-C₆; un dialkylamino en C₁-C₆; un cycloalkylamino en C₅-C₆; SH, SO₃H; SO₃ (alkyle en C₁-C₆) ; SO₂ (alkyle en C₁-C₆) ; SO₂NH₂; SO₂NH(alkyle en C₁-C₆) ; SO₂NH (arylalkyle en C₇-C₂₀); SO (alkyle en C₁-C₆) ; ou R₅ et R₆ forment ensemble un cycle phényle qui peut être non substitué ou substitué par un halogène, un nitro, un cyano, un thiocyanato; un alkyle en C₁-C₆; un trifluorométhyle; un alcoxy en C₁-C₆; CO₂H, CO(alkyle en C₁-C₆) ; un amino, un monoalkylamino en C₁-C₆, un dialkylamino en C₁-C₆, SH; SO₃H; H; SO₃ (alkyle en C₁-C₆) , SO₂(alkyle en C₁-C₆) , SO (alkyle en C₁-C₆) , et
X représente l'oxygène; le soufre, CH=CH ou NR₉ où R₉ est H, un alkyle en C₁-C₆, un alcényle en C₁-C₆, un arylalkyle en C₇-C₁₆,
dans lequel l'aryle est un aryle en C₆-C₁₀ et l'alkyle est un alkyle en C₁-C₆, un arylalcényle en C₇-C₁₆ où l'aryle est un aryle en C₆-C₁₀ et l'alcényle est un alcényle en C₁-C₆; un alcanoyle en C₁-C₆ et où l'aryle est non substitué ou mono- ou di- ou trisubstitué indépendamment avec un hydroxy, un halogène, un nitro, un cyano, un thiocyanato, un trifluorométhyle, un alkyle en C₁-C₃, un alcoxy en C₁-C₃, CO₂H, CO₂alkyle (en C₁-C₃) , CONH₂, CONH (alkyle en C₁-C₃) , CON(alkyle en C₁-C₃) , CO(alkyle en C₁-C₃), un amino, un (monoalkyle en Cᵢ-C₃) amino, un (dialkyle en C₁-C₃) amino, un cycloalkylamino en C₅-C₆, un (alcanoyle en C₁-C₃)amino, SH, SO₃H, SO₃ (alkyle en C₁-C₃), SO₂ (alkyle en C₁-C₃), SO(alkyle en C₁-C₃), un alkylthio en C₁-C₃ ou un alcanoylthio en C₁-C₃; et
étant entendu que quand R₂ est un hydroxy, R₃ ne peut être l'hydrogène, sauf quand R₄ est l'hydrogène, OCH₂OCH₃, OCH₂OC₂H₅ ou OC(Ph)₃ et des sels acceptables sur le plan pharmacologique, en vue de la préparation d'un médicament destiné au traitement et/ou la prévention de troubles susceptibles à l'inhibition de la calcineurine cible, où le trouble est le glaucome et/ou l'uvéite.

2. Utilisation selon la revendication 1, dans laquelle
R₁ est sélectionné parmi l'allyle, le cinnamyle, le cyclopropylméthyle ou le cyclobutylméthyle;
R₂ est sélectionné parmi les méthoxy, éthoxy, n-propyloxy, benzyloxy, benzyloxy substitué dans le cycle aromatique avec F, Cl, NO₂, CN, CF₃, CH₃ ou OCH₃; allyloxy, cinnamyloxy ou 3-phénylpropyloxy;
R₃ est sélectionné parmi l'hydrogène, le méthyle, l'éthyle, le benzyle ou l'allyle;
R₄ est sélectionné parmi l'hydroxy, le méthoxy, le méthoxyméthoxy ou l'acétyloxy;
R₅ et R₆ sont chacun et indépendamment sélectionnés parmi l'hydrogène; un nitro; un cyano, un chloro, un fluoro, un bromo, un trifluorométhyle; CO₂H; CO₂-CH₃; CONH₂; CONH-CH₃ ; SH; SO₂NH₂ ; N(CH₃)₂; SO₂CH₃; X est sélectionné parmi l'oxygène; NH ou NCH₃, N-benzyle, N-allyle.

3. Utilisation selon la revendication 1, dans laquelle le composé de formule (I) est sous forme d'un sel pharmaceutiquement acceptable.

4. Utilisation selon la revendication 1, dans laquelle le sel est un sel inorganique.

5. Utilisation selon la revendication 1, dans laquelle le sel est un sel organique.

6. Utilisation selon la revendication 1, dans laquelle le composé de formule (I) est
17-(cyclopropylméthyl)-6,7-déshydro-4,5α-époxy-14-éthoxy-3-hydroxy-5-méthyl-6,7-2',3'-indolomorphinan x HCl;
17-allyl-6,7-déshydro-4,5α-époxy-14-éthoxy-3-hydroxy-5-méthyl-6,7-2',3'-indolomorphinan x HCl;
6,7-déshydro-4,5α-époxy-14-éthoxy-3-hydroxy-5-méthyl-17-(2-phényl)éthyl-6,7- 2',3'-indolomorphinan x HCl;
17-allyl-6,7-déshydro-4,5α-époxy-3-hydroxy-14-méthoxy-5-méthyl-6,7-2',3'- indolomorphinan x HCl;
6,7-déshydro-4,5α-époxy-3-hydroxy-14-méthoxy-5-méthyl-17-(2-phényl)éthyl-6,7- 2',3'-indolomorphinan x HCl;
17-(cyclopropylméthyl)-6,7-déshydro-4,5α-époxy-3-hydroxy-14-méthoxy-5-méthyl-6,7-2',3'-indolomorphinan x HCl;
17-allyl-6,7-déshydro-4,5α-époxy-3-hydroxy-5-méthyl-14-n-propyloxy-6,7-2',3'- indolomorphinan x HCl;
17-(cyclopropylméthyl)-6,7-déshydro-4,5α-époxy-3-hydroxy-5-méthyl-14-n-propyloxy-6,7-2',3'-indolomorphinan x CH₃SO₃H;
17-(cyclopropylméthyl)-6,7-déshydro-14-(2',6'-dichlorobenzyloxy)-4,5α-époxy-3-(méthoxyméthoxy)-6,7-2',3'-benzo[b]furanomorphinan;
17-(cyclopropylméthyl)-6,7-déshydro-14-(2',6'-dichlorobenzyloxy)-4,5α-époxy-3-hydroxy-6,7-2',3'-benzo[b]furanomorphinan;
17-(cyclopropylméthyl)-6,7-déshydro-4,5α-époxy-3-(méthoxyméthoxy)-14-(3'-nitrobenzyloxy)-6,7-2',3'-benzo[b]furanomorphinan x HCl;
17-(cyclopropylméthyl)-6,7-déshydro-4,5α-époxy-3-hydroxy-14-(3'-nitrobenzyloxy)-6,7-2',3'-benzo[b]furanomorphinan x HCl;
17-(cyclopropylméthyl)-6,7-déshydro-4,5α-époxy-3-(méthoxyméthoxy)-14-(2'-naphthylméthoxy)-6,7-2',3'-benzo[b]furanomorphinan;
17-(cyclopropylméthyl)-6,7-déshydro-4,5α-époxy-3-hydroxy-14-(2'-naphthylméthoxy)-6,7-2',3'-benzo[b]furanomorphinan x HCl;
17-(cyclopropylméthyl)-6,7-déshydro-4,5α-époxy-14-(2'-fluorobenzyloxy)-3-(méthoxyméthoxy)-6,7-2',3'-benzo[b]furanomorphinan;
17-(cyclopropylméthyl)-6,7-déshydro-4,5α-époxy-14-(2'-fluorobenzyloxy)-3-hydroxy-6,7-2',3'-benzo[b]furanomorphinan x HCl;
14-cinnamyloxy-17-(cyclopropylméthyl)-6,7-déshydro-4,5α-époxy-3-(méthoxyméthoxy)-6,7-2'-3'-benzo[b]furanomorphinan;
salicylate de 14-cinnamyloxy-17-(cyclopropylméthyl) - 6,7-déshydro-4,5α-époxy-3-hydroxy-6,7-2'-3'-benzo[b]furanomorphinan;
17-(cyclopropylméthyl)-6,7-déshydro-4,5α-époxy-14-méthoxy-3-(méthoxyméthoxy)-6,7-2'-3'-benzo[b]furanomorphinan;
17-(cyclopropylméthyl)-14-(2'-chlorobenzyloxy)-6,7-déshydro-4,5α-époxy-3- (méthoxyméthoxy)-6,7-2',3'-(N-méthoxyméthylindolo)morphinan;
17-(cyclopropylméthyl)-14-(2'-chlorobenzyloxy)-6,7-déshydro-4,5α-époxy-3-hydroxy-6,7-2',3'-indolomorphinan x HCl;
17(cyclopropylméthyl)-6,7-déshydro-4,5α-époxy-3-hydroxy-14-(3'-chlorobenzyloxy)-6,7,2',3'-benzo[b]furanomorphinan x HCl;
17-(cyclopropylméthyl)-6,7-déshydro-4,5α-époxy-3-hydroxy-14-(2'-chlorobenzyloxy)-6,7,2',3'-benzo[b]furanomorphinan x HCl;
14-allyloxy-17-(cyclopropylméthyl)-6,7-déshydro-4,5α-époxy-3-hydroxy-1'-allyl-6,7-2',3'-indolomorphinan x HCl;
chlorhydrate de 17-(cyclobutylméthyl)-6,7-didéshydro-4,5α-époxy-14β-éthoxy-5β-méthylindolo[2',3':6,7]-morphinan-3-ol;
14β-(benzyloxy)-17-(cyclopropylméthyl)-6,7-didéshydro-4,5α-époxyindolo[2',3':6,7]morphinan-3-ol:
chlorhydrate de 14β-[(4-chlorobenzyl)oxy]-17-(cyclopropylméthyl)-6,7-didéshydro-4,5α-époxyindolo[2',3':6,7]morphinan-3-ol;
chlorhydrate de 17-(cyclopropylméthyl)-6,7-didéshydro-4,5α-époxy-14β-[(2-phénylbenzyl)oxy]indolo[2',3':6,7]-morphinan-3-ol;
chlorhydrate de 14β-[(4-t-butylbenzyl)oxy]-17-(cyclopropylméthyl)-6,7-didéshydro-4,5α-époxyindolo[2',3':6,7]morphinan-3-ol ;
17-(cyclopropylméthyl)-6,7-didéshydro-4,5α-époxy-14β-[(3-phénylpropyl)oxy]indolo[2',3':6,7]morphinan-3-ol.

7. Composé de formule (I) 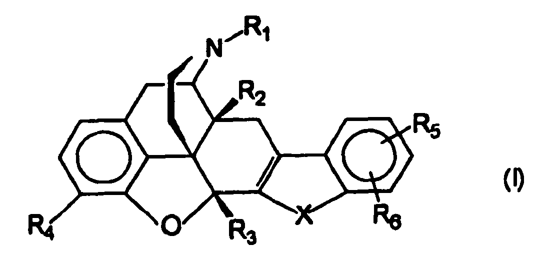 dans laquelle
R₁ représente un alcényle en C₁-C₁₀; un cycloalkylalkyle en C₄-C₁₀ où le cycloalkyle est un cycloalkyle en C₃-C₆ et l'alkyle est un alkyle en C₁-C₄; un cycloalcénylalkyle en C₄-C₁₀ où le cycloalcényle est un cycloalcényle en C₃-C₆ et l'alkyle est un alkyle en C₁-C₄; un arylalkyle en C₇-C₁₆ où l'aryle est un aryle en C₆-C₁₀ et l'alkyle est un alkyle en C₁-C₆; un arylalcényle en C₈-C₁₆ où l'aryle est un aryle en C₆-C₁₀ et l'alcényle est un alcényle en C₂-C₆;
R₂ représente l'hydrogène, un hydroxy, un alcoxy en C₁-C₆; un alcényloxy en C₁-C₆; un arylalkyloxy en C₇-C₁₆ où l'aryle est un aryle en C₆-C₁₀ et l'alkyloxy est un alkyloxy en C₁-C₆; un arylalcényloxy en C₇-C₁₆ où l'aryle est un aryle en C₆-C₁₀ et l'alcényloxy est un alcényloxy en C₁-C₆; un alcanoyloxy en C₁-C₆; un arylalcanoyloxy en C₇-C₁₆ où l'aryle est un aryle en C₆-C₁₀ et l'alcanoyloxy est un alcanoyloxy en C₁-C₆;
R₃ représente l'hydrogène, un alkyle en C₁-C₆; un alcényle en C₁-C₆; un arylalkyle en C₇-C₁₆ où l'aryle est un aryle en C₆-C₁₀ et l'alkyle est un alkyle en C₁-C₆; un arylalcényle en C₇-C₁₆ où l'aryle est un aryle en C₆-C₁₀ et l'alcényle est un alcényle en C₁-C₆; un hydroxyalkyle(en C₁-C₆); un alcoxyalkyle où l'alcoxy est un alcoxy en C₁-C₆ et l'alkyle est un alkyle en C₁-C₆; CO₂H; CO₂ (alkyle en C₁-C₆) ;
R₄ est l'hydrogène, un hydroxy; un alcoxy en C₁-C₆; un arylalkyloxy en C₇-C₁₆ où l'aryle est un aryle en C₆-C₁₀ et l'alkyloxy est un alkyloxy en C₁-C₆; un alcényloxy en C₁-C₆; un alcanoyloxy en C₁-C₆; un arylalcanoyloxy en C₇-C₁₆ où l'aryle est un aryle en C₆-C₁₀ et l'alcanoyloxy est un alcanoyloxy en C₁-C₆; un alkyloxyalcoxy en C₂-C₁₀ où l'alkyloxy est un alkyloxy en C₁-C₄ et l'alcoxy est un alcoxy en C₁-C₆;
R₅ et R₆ représentent chacun indépendamment l'hydrogène; OH; un alcoxy en C₁-C₆; un alkyle en C₁-C₆; un hydroxyalkyle où l'alkyle est un alkyle en C₁-C₆; un halogène; un nitro; un cyano; un thiocyanato; un trifluorométhyle; CO₂H; CO₂ (alkyle en C₁-C₆) ; CONH₂; CONH(alkyle en C₁-C₆); CON(alkyle en C₁-C₆)₂ ; un amino; un monoalkylamino en C₁-C₆; un dialkylamino en C₁-C₆; cycloalkylamino en C₅-C₆; SH; SO₃H; SO₃(alkyle en C₁-C₆); SO₂(alkyle en C₁-C₆); SO₂NH₂; SO₂NH(alkyle en C₁-C₆) ; SO₂NH (arylalkyle en C₇-C₂₀); SO(alkyle en C₁-C₆) ; ou R₅ et R₆ forment ensemble un cycle phényle qui peut être non substitué ou substitué par un halogène, un nitro, un cyano, un thiocyanato; un alkyle en C₁-C₆; un trifluorométhyle; un alcoxy en C₁-C₆, CO₂H, CO(alkyle en C₁-C₆), un amino, un monoalkylamino en C₁-C₆, un dialkylamino en C₁-C₆, SH; SO₃H; H; SO₃ (alkyle en C₁-C₆), SO₂(alkyle en C₁-C₆), SO (alkyle en C₁-C₆), et
X représente l'oxygène; le soufre, CH=CH ou NR₉ où R₉ est H, un alkyle en C₁-C₆, un alcényle en C₁-C₆, un arylalkyle en C₇-C₁₆,
dans lequel l'aryle est un aryle en C₆-C₁₀ et l'alkyle est un alkyle en C₁-C₆, un arylalcényle en C₇-C₁₆ où l'aryle est un aryle en C₆-C₁₀ et l'alcényle est un alcényle en C₁-C₆; un alcanoyle en C₁-C₆ et où l'aryle est non substitué ou mono- ou di- ou trisubstitué indépendamment avec un hydroxy, un halogène, un nitro, un cyano, un thiocyanato, un trifluorométhyle, un alkyle en C₁-C₃, un alcoxy en C₁-C₃, CO₂H, CO₂-alkyle(en C₁-C₃), CONH₂, CONH(alkyle en C₁-C₃), CON(alkyle en C₁-C₃), CO(alkyle en C₁-C₃), un amino, un (monoalkyle en C₁-C₃)amino, un (dialkyle en C₁-C₃)amino, un cycloalkylamino en C₅-C₆, un (alcanoyl en C₁-C₃)amino, SH, SO₃H, SO₃(alkyle en C₁-C₃), SO₂(alkyle en C₁-C₃), SO(alkyle en C₁-C₃), un alkylthio en C₁-C₃ ou un alcanoylthio en C₁-C₃; et
étant entendu que quand R₂ est un hydroxy, R₃ ne peut être l'hydrogène, sauf quand R₄ est l'hydrogène, OCH₂OCH₃, OCH₂OC₂H₅ ou OC(Ph)₃ et des sels acceptables sur le plan pharmacologique, en vue de l'utilisation dans le traitement et/ou la prévention de troubles susceptibles à l'inhibition de la calcineurine cible, dont le trouble est le glaucome et/ou l'uvéite.

8. Composé à utiliser selon la revendication 7,
dans lequel
R₁ est sélectionné parmi l'allyle, le cinnamyle, le cyclopropylméthyle ou le cyclobutylméthyle;
R₂ est sélectionné parmi les méthoxy, éthoxy, n-propyloxy, benzyloxy, benzyloxy substitué dans le cycle aromatique avec F, C1, NO₂, CN, CF₃, CH₃ ou OCH₃; allyloxy, cinnamyloxy ou 3-phénylpropyloxy;
R₃ est sélectionné parmi l'hydrogène, le méthyle, l'éthyle, le benzyle ou l'allyle;
R₄ est sélectionné parmi l'hydroxy, le méthoxy, le méthoxyméthoxy ou l'acétyloxy;
R₅ et R₆ sont chacun et indépendamment sélectionnés parmi l'hydrogène; un nitro; un cyano, un chloro, un fluoro, un bromo, un trifluorométhyle; CO₂H; CO₂CH₃; CONH₂; CONH CH₃ ; SH; SO₂NH₂ ; N(CH₃)₂; SO₂CH₃; X est sélectionné parmi l'oxygène; NH or N CH₃, N-benzyle, N-allyle.

9. Composé à utiliser selon la revendication 7,
dans lequel le composé de formule (I) est sous forme d'un sel pharmaceutiquement acceptable.

10. Composé à utiliser selon la revendication 7,
dans lequel le sel est un sel inorganique.

11. Composé à utiliser selon la revendication 7,
dans lequel le sel est un sel organique.

12. Composé à utiliser selon la revendication 7,
dans lequel le composé de formule (I) est
17-(cyclopropylméthyl)-6,7-déshydro-4,5α-époxy-14-éthoxy-3-hydroxy-5-méthyl-6,7-2',3'-indolomorphinan x HCl;
17-allyl-6,7-déshydro-4,5α-époxy-14-éthoxy-3-hydroxy-5-méthyl-6,7-2',3'-indolomorphinan x HCl;
6,7-déshydro-4,5α-époxy-14-éthoxy-3-hydroxy-5-méthyl-17-(2-phényl)éthyl-6,7-2',3'-indolomorphinan x HCl;
17-allyl-6,7-déshydro-4,5α-époxy-3-hydroxy-14-méthoxy-5-méthyl-6,7-2',3'-indolomorphinan x HCl;
6,7-déshydro-4,5α-époxy-3-hydroxy-14-méthoxy-5-méthyl-17-(2-phényl)éthyl-6,7-2',3'-indolomorphinan x HCl;
17-(cyclopropylméthyl)-6,7-déshydro-4,5α-époxy-3-hydroxy-14-méthoxy-5-méthyl-6,7-2',3'-indolomorphinan x HCl;
17-allyl-6,7-déshydro-4,5α-époxy-3-hydroxy-5-méthyl-14-n-propyloxy-6,7-2',3'-indolomorphinan x HCl;
17-(cyclopropylméthyl)-6,7-déshydro-4,5α-époxy-3-hydroxy-5-méthyl-14-n-propyloxy-6,7-2',3'-indolomorphinan x CH₃SO₃H;
17-(cyclopropylméthyl)-6,7-déshydro-14-(2',6'-dichlorobenzyloxy)-4,5a-époxy-3-(méthoxyméthoxy)-6,7-2',3'-benzo[b]furanomorphinan;
17-(cyclopropylméthyl)-6,7-déshydro-14-(2',6'-dichlorobenzyloxy)-4,5α-époxy-3-hydroxy-6,7-2',3'-benzo[b]furanomorphinan;
17-(cyclopropylméthyl)-6,7-déshydro-4,5α-époxy-3-(méthoxyméthoxy)-14-(3'-nitrobenzyloxy)-6,7-2',3'-benzo[b]furanomorphinan x HCl;
17-(cyclopropylméthyl)-6,7-déshydro-4,5α-époxy-3-hydroxy-14-(3'-nitrobenzyloxy)-6,7-2',3'-benzo[b]furanomorphinan x HCl;
17-(cyclopropylméthyl)-6,7-déshydro-4,5α-époxy-3-(méthoxyméthoxy)-14-(2'-naphthylméthoxy)-6,7-2',3'-benzo[b]furanomorphinan;
17-(cyclopropylméthyl)-6,7-déshydro-4,5α-époxy-3-hydroxy-14-(2'-naphthylméthoxy)-6,7-2',3'-benzo[b]furanomorphinan x HCl;
17-(cyclopropylméthyl)-6,7-déshydro-4,5α-époxy-14-(2'-fluorobenzyloxy)-3-(méthoxyméthoxy)-6,7-2',3'-benzo[b]furanomorphinan;
17-(cyclopropylméthyl)-6,7-déshydro-4,5α-époxy-14-(2'-fluorobenzyloxy)-3-hydroxy-6,7-2',3'-benzo[b]furanomorphinan x HCl;
14-cinnamyloxy-17-(cyclopropylméthyl)-6,7-déshydro-4,5α-époxy-3-(méthoxyméthoxy)-6,7-2'-3'-benzo[b]furanomorphinan;
salicylate de 14-cinnamyloxy-17-(cyclopropylméthyl)-6,7-déshydro-4,5α-époxy-3-hydroxy-6,7-2'-3'-benzo[b]furanomorphinan;
17-(cyclopropylméthyl)-6,7-déshydro-4,5α-époxy-14-méthoxy-3-(méthoxyméthoxy)-6,7-2'-3'-benzo[b]furanomorphinan;
17-(cyclopropylméthyl)-14-(2'-chlorobenzyloxy)-6,7-déshydro-4,5α-époxy-3-(méthoxyméthoxy)-6,7-2',3'-(N-méthoxyméthylindolo)morphinan;
17-(cyclopropylméthyl)-14-(2'-chlorobenzyloxy)-6,7-déshydro-4,5α-époxy-3-hydroxy-6,7-2',3'-indolomorphinan x HCl;
17-(cyclopropylméthyl)-6,7-déshydro-4,5α-époxy-3-hydroxy-14-(3'-chlorobenzyloxy)-6,7,2',3'-benzo[b]furanomorphinan x HCl;
17-(cyclopropylméthyl)-6,7-déshydro-4,5α-époxy-3-hydroxy-14-(2'-chlorobenzyloxy)-6,7,2',3'-benzo[b]furano-morphinan x HCl;
14-allyloxy-17-(cyclopropylméthyl)-6,7-déshydro-4,5α-époxy-3-hydroxy-l'-allyl-6,7-2',3'-indolomorphinan x HCl;
chlorhydrate de 17-(cyclobutylméthyl)-6,7-didéshydro-4,5a-époxy-14β-éthoxy-5β-méthylindolo[2',3':6,7]-morphinan-3-ol;
14β-(benzyloxy)-17-(cyclopropylméthyl)-6,7-didéshydro4,5α-époxyindolo[2',3':6,7]morphinan-3-ol:
chlorhydrate de 14β-[(4-chlorobenzyl)oxy]-17-(cyclopropylméthyl)-6,7-didéshydro-4,5α-époxyindolo[2',3':6,7]morphinan-3-ol;
chlorhydrate de 17-(cyclopropylméthyl)-6,7-didéshydro-4,5α-époxy-14β-[(2-phénylbenzyl)oxy]indolo[2',3':6,7]-morphinan-3-ol;
chlorhydrate de 14β-[(4-t-butylbenzyl)oxy]-17-(cyclopropylméthyl)-6,7-didéshydro-4,5α-époxyindolo[2',3':6,7]morphinan-3-ol;
17-(cyclopropylméthyl)-6,7-didéshydro-4,5α-époxy-14β-[(3-phénylpropyl)oxy]indolo[2',3':6,7]morphinan-3-ol.
